# EUROPEAN PATENT SPECIFICATION

(11) **EP 4 355 401 B1**
(45) Date of publication and mention of the grant of the patent: **19.11.2025**
(21) Application number: 22734563.4
(22) Date of filing: 14.06.2022
(51) Int. Cl.: A61M 25/00, A61M 25/06

(54) **NEEDLE DEVICES WITH GRIP ADAPTORS AND RELATED METHOD**
NADELVORRICHTUNGEN MIT GRIFFADAPTERN UND ZUGEHÖRIGES VERFAHREN
DISPOSITIFS À AIGUILLE DOTÉS D'ADAPTATEURS DE PRÉHENSION ET PROCÉDÉ ASSOCIÉ

(30) Priority: 14.06.2021 US 202163210352 P
(43) Date of publication of application: 24.04.2024
(73) Proprietor: B. Braun Melsungen AG, 34212 Melsungen (DE)
(72) Inventor: Kavintharan, Penang, 10810 (MY); MARZUKI, Siti Nurulhidayah, Penang, 10810 (MY); MOHD MAHADZIR, Mimi Mawar Atikah, Penang, 10810 (MY)
(74) Representative: Kinkeldey, Daniela
(86) International application number: PCT/EP2022/066135
(87) International publication number: WO 2022/263427

(56) References cited:
- WO-A1-2021/038041
- US-A- 4 445 893
- US-A1- 2009 105 689
- US-A1- 2017 120 009
- US-A1- 2020 001 050

## Description

### FIELD OF ART

The present disclosure is generally related to needle devices with specific discussions on needle devices, such as catheter assemblies, with grip adaptors and hub extensions and related method.

### BACKGROUND

Insertion procedure for an IV catheter assembly contains four basic steps: (1) the healthcare worker inserts the needle and catheter together into the patient's vein; (2) after insertion into the vein with the needle point, the catheter is forwarded into the vein of the patient by the healthcare worker pushing the catheter with his or her finger; (3) the healthcare worker withdraws the needle by grasping the hub end (opposite the point end) while at the same time applying pressure to the patient's skin at the insertion site with his or her free hand to slow down or stop the flow of blood through the catheter; and (4) the healthcare worker then tapes the exposed end of the catheter (the catheter hub) to the patient's skin and connects it to the source of the fluid to be administered into the patient's vein.

The problem is that immediately after the withdrawal of the needle from the patient's vein, the healthcare worker, who is at this time involved in at least two urgent procedures, must place the exposed needle tip at a nearby location and address the tasks required to accomplish the needle withdrawal. It is at this juncture that the exposed needle tip creates a danger of an accidental needle stick, which, under the circumstances, leaves the healthcare worker vulnerable to the transmission of various dangerous blood-borne pathogens, including AIDS and hepatitis.

Other needle types similarly expose healthcare workers to risks of accidental needle sticks. For example, a doctor administering an injection, using a straight needle, a Huber needle, an epidural needle, etc., may place the used needle on a tray for subsequent disposal by a nurse. During the period between placing the used needle on a tray or a workstation to the time it is discarded, the used needle is a potential source for disease transmissions for those that work near or around the needle.

Accordingly, exposed needle tips should be covered immediately following use to ensure greater worker safety. Ideally, the procedure for covering the needle tip should be passive, self-activating, or at least simple to perform. In addition, the device for covering the needle should be reliable and robust.

Needle devices often include safety systems that cover the tip of the needle to prevent accidental sticks after placement of the catheter tube into the vasculature of a patient. These systems can be either passive or active. In some systems, the safety features are located inside the catheter hub in the ready position while in other systems they are external of the catheter hub. In either location, the safety features serve the same function, to cover the needle tip in order to prevent accidental needle sticks after venipuncture.

US 2009/0105689 A1 discloses a removable push-off tab for IV catheter.

US 2017/0120009 A1 discloses soft push tabs for catheter adapter.

WO 2021/038041 A1 discloses extended dwell and midline catheters and related methods.

US 4,445,893 A discloses an infusion apparatus.

US 2020/0001050 A1 discloses a medical device with anti-rotation push tab.

### SUMMARY

The invention is determined by the subject-matter of the independent claims. Further aspects are defined in dependent claims 2-18.

Aspects of the present invention comprises a catheter assembly comprising a catheter unit and a needle hub with a needle.

The catheter unit can comprise a catheter hub with a catheter tube, a hub extension, and a tab adaptor. In an example, the tab adaptor can be removable from the catheter hub.

The catheter hub can have a hub body with an interior cavity and a needle guard can be located in the interior cavity.

The catheter hub can have a hub body with an interior cavity having a valve and a valve opener located in the interior cavity.

Aspects of the invention further can include a catheter assembly comprising: a catheter hub comprising a hub body with a width and a saddle socket having wall surfaces defining a width that is wider than the width of the hub body, the hub body having an upper surface located, elevation-wise, above a lower surface; a catheter tube having a length and a lumen extending distally of a nose section of the catheter hub; a needle attached to a needle hub and having a needle tip extending through the hub body and the lumen of the catheter tube in a ready to use position; a hub extension having a body with a pocket having at least part of the hub body and the saddle socket located in the pocket and constrained from moving relative to the hub extension by the body; and a tab adaptor surrounding at least part of the upper surface of the catheter hub, the tab adaptor having two sidewalls defining a width that is wider than the width of the hub body, wherein the tab adaptor is separable from the catheter hub and wherein the saddle socket is attached to the hub body and the tap adaptor is attached to the saddle socket.

The hub extension can comprise a pair of wings extending laterally of the pocket.

A tip having a lumen can be provided with the hub extension, and wherein at least part of the nose section of the catheter hub can be located in the lumen.

The hub extension can have a flat bottom surface. The flat bottom surface can extend from a distal end to a proximal end.

The proximal end of the hub extension can have a straight trailing edge.

The hub extension can be made from a material that is softer and more flexible than a material used to form the hub body of the catheter hub.

The tab adaptor can have a lower surface and two spaced apart anchors extending from the lower surface.

The socket saddle can have two spaced apart sockets and wherein the two anchors can engage the two sockets.

The pocket can have a bottom wall comprising a plurality of mating members.

The saddle socket can comprise a plurality of mating members, and wherein the mating members at the bottom wall of the pocket can engage the mating members on the saddle socket.

A needle guard can be located in an interior cavity of the hub body of the catheter hub.

A valve and a valve opener can be located in an interior cavity of the catheter hub.

The hub extension can have a leading edge and a trailing edge that are not parallel.

The hub extension can have a D-shaped configuration with a projection extending axially of a curved section of the D-shaped configuration. The D-shaped configuration can be ascertained from a top plan view or a bottom plan view.

The hub extension can have two side edges defining a maximum width of the hub extension.

The maximum width of the hub extension can be wider than the width of the tab adaptor and the width the hub body.

The catheter hub can have a push tab and the tab adaptor can have a recessed section at a lower surface located around the push tab.

The tab adaptor can have a concave surface located between the two sidewalls.

The hub extension can have a tip comprising a distal opening and a proximal opening, and wherein the distal opening can have a ramped surface and the proximal opening can have the nose section of the catheter hub located therein.

Further disclosed is an illustrative example of a catheter assembly (not claimed) comprising: a catheter hub comprising a hub body with a nose section at a distal end, a female Luer at a proximal end, and a lengthwise axis extending between the nose section and the female Luer; a catheter tube having a length and a lumen extending distally of the nose section of the catheter hub; a needle attached to a needle hub and having a needle tip extending through the hub body and the lumen of the catheter tube in a ready to use position; a hub extension having a body with a bottom surface that is planar and a pocket having at least part of the hub body of the catheter hub located in the pocket such that the hub body is constrained from moving relative to the hub extension and the lengthwise axis is angled to the bottom surface; and a tab adaptor surrounding at least part of the upper surface of the catheter hub, the tab adaptor having sidewalls defining a width that is wider than a width of the hub body.

Aspects of the invention further include a method of manufacturing a catheter assembly. The method comprises forming a catheter hub comprising a hub body with a width and a saddle socket having wall surfaces defining a width that is wider than the width of the hub body, the hub body having an upper surface located, elevation-wise, above a lower surface; attaching a catheter tube having a length and a lumen to a nose section of the catheter hub; extending a needle attached to a needle hub and having a needle tip through the hub body and the lumen of the catheter tube in a ready to use position; attaching a hub extension having a body with a pocket to the catheter hub by placing at least part of the hub body and the saddle socket in the pocket and constraining the catheter hub from moving relative to the hub extension; and placing a tab adaptor over at least part of the upper surface of the catheter hub, the tab adaptor having two sidewalls defining a width that is wider than the width of the hub body, wherein the tab adaptor is separable from the catheter hub, the saddle socket is attached to the hub body and the tap adaptor is attached to the saddle socket.

A method of using any of the foregoing catheter assemblies is not part of claimed invention and presented for illustrative purposes.

A needle assembly or needle device provided in accordance with aspects of the present disclosure can comprise a first hub having an over-the-needle tube or tubing attached thereto and a second hub having a needle attached thereto. The tubing has a lumen or bore for receiving the needle and has a distal opening or distal end opening.

The needle assembly can be called a catheter assembly or a catheter device. The needle device or assembly is configured for peripheral access but components used therein can apply to other or different applications.

The needle has a needle tip and can include a change in profile located proximal of the tip for use with a needle guard. The needle tip extends distally of the distal end opening of the tube or tubing in the ready to use position with the change in profile, if incorporated for use with a needle guard, located proximally of the distal tube opening. The change in profile can be a crimp, a bump, or a material build-up having different profile than other diameter sections of the needle shaft.

As used herein, the term proximal is understood to mean an end or side closer to the practitioner and the term distal is the opposite end or side.

The change in profile can be used to interact with a needle guard during retraction of the needle. The first hub may alternatively be referred to as a catheter hub and the second hub may alternatively be referred to as a needle hub. A vent plug is disposed at the proximal open end of the second hub and has a vent filter at a proximal end thereof, which is conventional. The tube is attached to the first hub by a ferrule or metal bushing and may be referred to as a catheter tube.

A needle guard can optionally be provided in the interior cavity of the first hub for covering the needle tip in the protective position, such as following successful venipuncture. In some examples, the needle guard can be omitted. If so, the change in profile can also be omitted. In still other examples, a needle guard can be included without also including a change in profile on the needle. For example, the needle guard can have a perimeter defining an opening and the opening can cant or lean over when activated to grip the needle. In yet other examples, the needle guard 120 can be located in a third housing between the first hub and the second hub.

The needle guard can include a proximal wall and two arms extending distally of the proximal wall. The two arms can intersect one another in the ready to use position and in the protective position in which the needle guard covers the needle tip, and as viewed from a side. In some examples, the two arms of the needle guard can extend in a distal direction without intersecting one another. Two distal walls, one on each arm, can be incorporated to block the needle tip.

The two distal walls can bias outwardly by the needle in the ready to use position and disposed distally of an internal change in profile inside the first hub in the ready to use position. The internal change in profile can be a reduced diameter section located next to an inside diameter section of a larger dimension. Each distal wall can include a curved lip to facilitate relative movement between the needle guard and the needle. The two distal walls can also each have a curved lip at an end thereof to improve sliding movement along the needle shaft.

When in the ready to use position, the dimension measured between the two joints or elbows at each arm, at the intersection between each arm and its respective distal wall, can be larger than the inside diameter of the internal change in profile, which prevents the needle guard from moving proximally thereof. Once the needle tip moves proximally of the two distal walls and the arms no longer biased by the needle shaft, the two arms move radially inward and one or both distal walls close over the needle tip to block the needle tip in a protective position. At that point, the dimension between the two joints or elbows decreases and is smaller than the internal dimension of the internal change in profile, which then allows the needle guard to move proximally and be removed from the catheter hub with the needle.

The first hub has a proximal opening having a nose section of the second hub disposed therein. The proximal opening has a female Luer for receiving a male Luer tip, such as a syringe, an IV tubing adaptor, a Luer extension, etc. External threads can be provided on the exterior surface at the proximal end for threaded engagement with a threaded collar of a male Luer tip.

A pair of stabilizing wings can extend radially of the first hub to facilitate securement or anchoring of the first hub to a patient following successful venipuncture. Optionally the wings can be omitted. The first hub can embody a standard IV catheter hub without a tubing port. In other examples, the first hub can include a tubing port and a tubing connected thereto, with a needleless valve connector connected on the opposite end of the tubing, which is also known as an integrated catheter. In still other examples, the first hub can include a side fluid port, also known as a ported catheter.

Externally, a push tab can be unitarily formed with the hub body. The push tab can be used by a practitioner to grip and advance the needle device, such as to gain peripheral access. The push tab allows a practitioner to make adjustments on the placement, location or movement of the catheter hub.

The first hub can be removably secured to the second hub by receiving the nose section of the second hub in the proximal opening of the first hub. A flange or extension can be provided on the second hub and together with the nose section define a gap having part of the first hub located therein. Optionally the flange or extension can be omitted and a stub or other surface mating features provided. The stub can limit the extent of insertion of the second hub into the first hub. A rib or annular flange can also extend from the nose section of the second hub to limit the extent of insertion of the nose section into the first hub.

The second hub has a body having an interior cavity having the proximal end of the needle projecting there-into. The interior cavity can function as a primary blood flashback chamber. The proximal opening of the body can have a female Luer for receiving a vent plug therein and the exterior can have threads or be without threads. In an example, the exterior of the body of the second hub is generally cylindrical and without threads for receiving an adjustable grip or extension clip, as further discussed below. The first and second hubs can be made from plastic materials, such as by plastic injection.

Internally of the catheter hub, the catheter hub may include a valve and a valve opener located in the interior of the catheter hub, optionally also with a needle guard. The valve can comprise a plurality of slits defining a plurality of flaps and the valve opener can comprise a nose section and one or more actuating elements extending in the proximal direction from the nose section. The valve opener can be pushed in a distal direction by a male Luer tip, such as a syringe tip, to open the valve flaps. Catheter assemblies with a needle guard, a valve, and a valve opener that are usable with catheter assemblies of the present application are disclosed in U.S. Publication No. 2018/0214682 A1 to Woehr et al. and in U.S. Publication No. 2020/0188634 A1 to Woehr et al..

The present needle device 100 can comprise a catheter hub body having an interior, which can accommodate at least one of a needle guard, a valve, and a valve opener. The catheter tube can attach to the distal end of the hub body with a metal bushing or ferrule, which is standard or traditional. The hub body further comprising an exterior having an upper surface located above, elevation-wise, a lower surface.

A hub extension and a push tab adaptor can be used with a catheter hub. The hub extension can include a body having a receiving space that is sized and shaped to receive the catheter hub body. The hub body typically has a body that is rounded or not substantially flat, which can shift or rotate during use. The hub extension can provide an enlarged surface area so that when the hub extension is secured to the patient following successful venipuncture, the enlarged surface area provides comfort and stability from rotating, such as during connector change.

In an example, the push tab adaptor can be removably attached to the hub body, such as by interference, tapered fit, friction fit, snap fit, detents, or equivalent attachment means or connection means. In other examples, the tab adaptor can be removably attached to the hub extension. A typical unitarily formed push tab has a first size that is configured for pushing or manipulating when using the catheter assembly. The push tab adaptor of the present embodiment provides enlarged gripping surfaces above and beyond the first size, i.e., enlarged to a second larger size, for a practitioner to grip and push, and/or pull, the needle assembly when performing vascular access. The second size being larger than the first size.

The enlarged gripping surfaces provides stability, improved access to gripping, and less prone to rotation during use. The push tab adaptor can engage the hub body or alternatively can engage the hub extension for use during vascular access so that a relatively larger surface is made available for the user to use during insertion of the needle and catheter. The enlarged push tab adaptor provides a large surface for the user to grip and manipulate and as well as shield the catheter hub body from direct contact by the user by serving as the intermediary. The push tab adaptor can subsequently be removed following successful venipuncture. The enlarged surface can increase the width, the height, or both the width and the height of the unitarily formed push tab.

The enlarged surface of the push tab adaptor provides stability, improved access to gripping, and less prone to rotation during use while at the same time can be removed so that tenting from placing an adhesive dressing over the catheter hub, without the tab adaptor, can be minimized.

The combination catheter hub, hub extension, tab adaptor, and catheter tube attached to the catheter hub may herein be referred to as a catheter unit.

The hub extension can have a body comprising a first end or distal end and a second end or proximal end, which define a length therebetween. In an example, the hub extension has a tip comprising a length that is shorter than the length of the hub extension. The tip has a distal end with a distal opening and a proximal end with a proximal opening and a lumen extending between the two openings.

The distal end of the tip of the hub extension can have a ramped surface. The ramped surface can resemble a truncated frustoconical structure, such as a truncated cone structure, with a relatively thin leading edge. This ramped surface can allow the catheter tubing to form a controlled bend, or provides a surface that supports the catheter tube, when in use so as to avoid kinking.

The proximal opening of the tip of the hub extension can be sized and shaped to receive the nose section of the catheter hub. In an example, the proximal opening can have corresponding negative surfaces or complementary surfaces as the nose section of the catheter hub for receiving the nose section of the catheter hub.

The bore or lumen of the tip of the hub extension can be sized and shaped to accommodate the catheter tube. Thus, part of the nose section and catheter tube can both be located in the lumen of the tip. The clearance or tolerance between the lumen and the catheter tube should be a loose fit or a size-on-size fit. The tip can have a generally cylindrical exterior. The tip can alternatively have a generally polygonal exterior with chamfered or rounded corners connecting the sides. The exterior surfaces of the tip can be smooth or can be roughened, such as having textures, or both.

The body of the hub extension can include a cavity or pocket for engaging or receiving part of the hub body of the catheter hub, with part of the proximal cylinder, including the Luer threads, of the catheter hub extending proximally of the second end of the hub extension. The amount of the catheter hub that extends proximally of the second end can vary.

In some examples, the catheter hub can incorporate a pocket and the hub extension can include a projection that projects into the pocket of the catheter hub to secure the two components together.

A pair of wings can extend laterally of the length of the body of the hub extension, or laterally of the hub body. Each of the two wings can have a leading edge, a trailing edge and an apex or connecting portion that connects the two edges. In an example, the leading edge can be curved or arcuate while the trailing edge can be generally straight.

Starting from about the proximal end of the tip, each of the two wings of the hub extension can be angled in the proximal direction and curved as it extends in the proximal direction. An embodiment of the present invention includes trailing edges for the two wings that generally align, such as in a generally straight line. In a particular example, the two trailing edges of the two wings form a generally straight line or edge with a proximal edge of the central body portion.

From a bottom view, the hub extension resembles a D-shape, along a top view or bottom view, with a stub projection extending from the curved part of the D-shape. The hub extension provides a generally flat surface that spans from the nose section of the catheter hub along the length of the catheter hub to about a mid-length of the catheter hub so that the curved or rounded body of the catheter hub does not directly contact the patient.

In alternative embodiments, the trailing edges, and possibly the proximal edge of the central body portion, can include some non-linear or curved sections. For example, the entire proximal edge of the body can bow outwardly in the proximal direction to further increase the size of the bottom surface.

Each wing can have a base portion having a curved or sloped surface. In the catheter unit assembled configuration, the generally cylindrical hub portion of the catheter hub body and the sloped base portion can have a dividing or split line therebetween, formed by snapping the catheter hub into the pocket or cavity of the hub extension.

The two base portions of the two wings and the upper portion of the catheter hub body can form a generally continuous smooth hump, with some change in inflection possible. Said differently, the curvature of the catheter hub body and the sloped base portion can form a smooth transition that gradually decreases the height of the catheter unit as it extends towards the tip or apex of each wing. Thus, when the tab adaptor is removed following vascular access, as further discussed below, the remaining structure has a smooth contour that facilitates securement of the adhesive dressing.

The tab adaptor can be located over or on top of the catheter hub body. The tab adaptor can be removably attached to the catheter hub body, as further discussed below. In other examples, the tab adaptor can be removably attach to the hub extension, such as via detents, snap fit, interference fit or combinations thereof.

In an embodiment, the tab adaptor has a body with a width and wherein the width of the body is wider than the width of the cylindrical catheter hub body. In an example, the width of the catheter hub is X, and the width of the tab adaptor is 1.1X to 2.5X, and more preferably from 1. 5X to 2.2X the width of the catheter hub. The enlarged width and height of the tab adaptor provide stability and a large surface to grab, grip, or contact by the user so as to minimize directly contacting the catheter hub body and presents a stable surface for use during vascular access.

The body of the tab adaptor can have two opposed sidewalls and a concave surface or dimple located between the two sidewalls and presenting a contoured surface for pushing or pulling. The two sidewalls can each have the shape of a sail or shark dorsal fin. The curved part of each of the two sidewalls can orient in the proximal direction while the more vertical or straight part of the sidewall faces the distal direction. Thus, each sidewall has a straight part, a curved part, and a connecting part connecting the straight part and the curved part.

The concave surface provides a cradle or seat for the finger or fingers for improving finger grip when using the tab adaptor to advance the catheter assembly. In some examples, textured features, such as bumps, small recessed dimples or fins, maybe included on surfaces of the tab adaptor, such as the concave surface, to provide added gripping features.

The concave surface can also slope downward in the proximal direction. The sloping surface allows the user to push the tab in the distal direction with better leverage during vascular access. Alternatively, the user can use the crest at the upper distal end of the concave surface and the distal wall of the tab adaptor to wedge a finger against and pull the catheter assembly when performing vascular access. In some examples, the concave surface can extend proximally but not sloped as it extends proximally. In alternative embodiments, the surface between the two sidewalls can sloped downwardly in the proximal direction but can be flat or planar as it slopes.

A crest or ridge can form at the intersection of the concave surface and the distal wall, between the two sidewalls. In an example, the crest is arc shape. The center of the crest can be below, elevation-wise, the highest points on the two sidewalls. Thus, a finger placed at the crest is cradled or flanked by the surrounding surfaces to provide stability and grip. The distal wall is convex and bulges distally.

In some examples, the distal wall can be flat or planar or even concave. In an example, the proximal wall of the tab adaptor is generally flat or planar. The proximal wall can align with the trailing edge of the hub extension. Optionally, the proximal wall can bulge proximally to present additional surfaces for gripping.

In an example, the hub extension can be made from a soft thermoplastic material or a soft thermoplastic elastomer (TPE) material, having a softer feel than the material used to form the catheter hub 102. Preferably, the soft thermoplastic material or the soft TPE material is soft and flexible. Preferably, the soft thermoplastic material or the soft TPE material is flexible and at least the bottom surface of the hub extension is smooth for improved patient comfort. That is, the bottom surface can be generally flat, and the flat surface can extend from the tip to the proximal end, such as to the trailing edges of the wings. The wide and general flat bottom surface provide a stable base to prevent rotation of the catheter hub, such as during use, during patient movement, and during connector change out, which in turn improves patient comfort. The enlarged and general flat bottom surface distribute forces from being concentrated at any uneven point on the catheter unit that may rise or extend above other points or surfaces on the hub extension. With this, the pressure is equally distributed to prevent discomfort, pressure ulcers and pressure markings. In some examples, one or more recessed channels can be provided at the bottom surface to provide ventilations.

The soft material used to form the hub extension can be pliable and can partially conform to the patient for added patient comfort. In an example, the catheter hub can be made from a polyurethane material. The catheter hub may alternatively be made from a polypropylene (PP) material with other conventional materials for forming catheter hub contemplated.

The hub extension can be made from low density polyethylene (LDPE), polyethylene terephthalate (PET), or polyvinyl chloride (PVC), or equivalents. Alternatively, the hub extension can be made from a TPE material, such as thermoplastic olefins, styrene SBS (styrene-butadiene-styrene block copolymer), SEBS (styrene-ethylene-butylene-styrene block copolymer) or SEPS (styrene-ethylene-propylene-styrene block copolymer) compound, vulcanized PP/EPDM (ethylene propylene diene monomer rubber) compound, thermoplastic polyurethane, or thermoplastic polyamide. Other non-limiting examples include polycarbonate (PC), glass filled nylon, PP, high density polyethylene (HDPE), polystyrene, and acrylonitrile butadiene styrene (ABS).

The catheter hub can have a lengthwise axis extending from the distal end to the proximal end of the hub body. A standard prior art catheter hub, when placed on a flat planar surface, typically has a lengthwise axis that is approximately parallel to the flat surface. In the present embodiment, the lengthwise axis of the catheter hub is angled to the flat surface, such as angled to the ground or to the flat bottom surface of the hub extension, by about 1 degree to about 15 degrees with 3 to 8 degrees being more preferred. The angled configuration can be arranged by adjusting how the catheter hub is positioned relative to the hub extension 18 and the hub extension suspending the catheter hub in a way that the lengthwise axis is angled relative to the flat surface. The angled configuration allows the distal tip of the hub extension to be angled closer to a surface, such as to a patient's skin, to minimize or eliminate potential catheter tube kinking.

The separated position of the catheter unit is analogous to when vascular access or successful venipuncture has been achieved and the tab adaptor is separated from the catheter hub before the catheter hub is secured to the patient, such as by adhesive or medical dressing. The catheter hub has a unitarily formed push tab. The push tab has a height and a width, which can be standard or conventional. In an example, the height of the unitarily formed push tab can be shorter or smaller than traditional push tabs formed with traditional catheter hubs. The push tab enables the practitioner to adjust or manipulate the catheter hub should the tab adaptor be removed.

When a relatively shorter and/or smaller push tab is incorporated, the issue of tenting is reduced. Tenting arises when a tape, adhesive or dressing is placed over the catheter hub and the projection of the push tab, rising above the exterior surface of the hub body, creates un-even and non-contacting surfaces with the adhesive, which in turn traps air pockets and reduces effectiveness of the dressing adhesion. In some examples, the unitarily formed push tab can be omitted from the catheter hub as the removable tab adaptor can serve as a structure for pushing or manipulating the catheter hub without also utilizing the unitarily formed push tab.

In an example, the tab adaptor has two anchors extending downwardly from the body. The two anchors can be spaced from one another. Each of the two anchors can locate below, elevation-wise, the lower surface of the tab adaptor and generally aligns with a corresponding sidewall. In some examples, each anchor can recess from a side edge or sidewall of the tab adaptor. The two anchors can be substantially similar or identical. Each anchor can comprise a height and a width. Each anchor can comprise an extended surface and a radial surface. The two surfaces of the anchor can resemble a barb or a male detent. In other examples, each anchor resembles a stub.

The far end of each anchor, remote from the bottom of the tab adaptor, can have a greater thickness, measure orthogonally to the extended surface, than near the upper end adjacent the bottom of the tab adaptor. This arrangement minimizes potential for trapping air when the anchors are connected to the hub body. In other examples, gaps or channels between surfaces of the anchors and the receiving sockets of the catheter hub can be incorporated to minimize air trap.

The catheter hub of the present invention can comprise a receiving socket located laterally of the lengthwise axis of the catheter hub. Preferably, a receiving is provided on either side of the lengthwise axis of the catheter hub. The catheter hub with one or two sockets can have a catheter tube attached thereto and extending in a distal direction and a proximal opening with a female Luer.

Each socket can be sized and shaped to receive a corresponding anchor extending at the bottom of the tab adaptor. In some examples, the receiving socket can have a bottom that is deeper than the reach of the anchor. This extra space can be provided to incorporate a closure system for closing the opening of the receiving socket when the anchor is removed, as further discussed below.

In an example, each socket can have a perimeter defining an opening. The perimeter can have multiple sides, or have a polygonal configuration. In other examples, the perimeter can have curved edges and can have an irregular shape. The perimeter and the anchor can have corresponding shapes so that the anchor can project into the receiving socket. As shown, the perimeter of the socket defines a plane and wherein the plane of the receiving socket is slanted or angled relative to a horizontal plane, which can be the ground, the patient's skin, or the plane defined by the bottom surface of the hub extension.

The slant can be downward as the opening extends radially of the lengthwise axis. In other examples, the plane can be horizontal or even slants upwardly as it extends radially of the lengthwise axis. The downward slant of the opening can generally coincide, matches, or aligns with the slanted or curved profile of the base portion of the wing. This shape or configuration provides a smooth transition between the catheter hub and the hub extension when the tab adaptor is removed, which improves bonding with the adhesive dressing and reduces the number and size of potential air pockets.

The receiving socket of the catheter hub can have an upper opening, two sidewalls, and a bottom wall. The receiving socket can also have two end walls at two ends of the sidewalls. The various walls define a socket for receiving the anchor. The various walls can be generally orthogonal to one another and intersections between the walls can be radiused. The upper opening and the bottom wall define a receiving socket depth, which can be variable because of the angled upper opening. The receiving socket depth can have the same but negative or complementary size as the anchor.

Alternatively, the receiving socket depth can be deeper than the length of the anchor. The size difference can serve as space for incorporating additional features, such as a foam, biasing member, or helical compression spring to aid in pushing the anchor upwardly to assist with removal of the tab adaptor and/or to deploy a cover for covering the upper opening following removal of the tab adaptor. In an example, a pliable sleeve, such as a thin rubber sleeve is provided over a compression spring and the combination is located in the receiving socket. As the anchor is inserted into the receiving socket, the anchor can compress the spring and the sleeve. When the anchor is removed, the spring can expand and pushes the sleeve upward to cover the upper opening of the receiving socket.

A rotatable or bendable flap or a flap that swings can be provided at the upper opening of the receiving socket. The flap can be unitarily formed with the body of the hub extension, or can be attached to the body, such as by adhesive or by welding. When incorporated, the size of the receiving socket can be adjusted accordingly to accommodate both the flap and the anchor. The flap can swing inwardly into the interior space of the receiving socket when the anchor is inserted and can swing upwardly to at least partially cover the opening when the anchor is removed.

As shown, the tab adaptor can have a lower surface that is curved. The lower surface and the curvature of the hub body of the catheter hub can be complementary so that when the tab adaptor is removably secured to the catheter hub, the two surfaces are formed fitting. In some examples, the anchor and the sidewall of the tab adaptor have coplanar surfaces. However, the anchor can recess from the sidewall and a lip can extend radially of the anchor. The lip can abut or contact the perimeter of the upper opening or the wing when the tab adaptor is secured to the hub.

An alternative receiving socket can include a pliable or biasing unit located in the interior of the receiving socket. The biasing unit can be a combination of a compression spring and a sleeve, can alternatively be a spring and a telescoping structure, or can alternatively be a porous foam or sponge material. The biasing unit can compress upon being pushed by the anchor and can expand upon removal of the anchor to cover at least part of the upper opening of the receiving socket.

In an example, the walls of the receiving socket can be provided with gripping features, such as elastomeric layers or bumps, to grip the anchor when assembling the tab adaptor to the catheter hub. Alternatively, a gripping sleeve may be provided or secured around the anchor and the gripping sleeve and anchor may be inserted into the receiving socket as a unit to assemble the tab adaptor to the catheter hub. The gripping sleeve can be made from any number of rubbery tacky material to increase friction between the sleeve and the wall surfaces of the receiving socket. Still alternatively, the tab adaptor can incorporate a receiving socket and the catheter hub can incorporate a complementary projection for projecting into the socket of the tab adaptor. This arrangement eliminates any pocket that may collect fluids and dust, etc. The tab adaptor can incorporate two receiving sockets or one receiving socket and one anchor.

The catheter hub described herein can have a hub body having a generally cylindrical main body section, which is conventional. A unitarily formed push tab can be provided with the body section. At the distal end of the hub body, a nose section can be located having a relatively smaller physical profile than the generally cylindrical main body section. In an example, the nose section can have a distal section, a mid-section, and a base section. In an example, the distal section can be generally cylindrical, the mid-section can be frusto-conical in shape, and the base section can be cylindrical or frusto-conical in shape.

In an example, the base section has distinct sides, such as being polygonal in shape. The base section can have four sides with each side having arcuate or curved sub-sections. In other examples, the sides can be straight and can connect to one another via curved sections. Alternatively, the base section can be circular or cylindrical in shape. The nose section can be configured to project into the proximal opening of the tip of the hub extension. In an example, the bore of the tip of the hub extension and the nose section of the catheter hub can have complementary surfaces. The complementary surfaces can facilitate alignment and secures the distal end of the catheter hub to the hub extension, such as by physical constraint.

The catheter hub can be constrained from moving relative to the hub extension by the body of the hub extension from the ready to use position and through the used position, such as being constrained from moving axially within the pocket of the hub extension. The nose section can press against the interior surface of the tip to limit looseness or slack. The shaped base section of the catheter hub can rest against complementary surfaces formed in the distal pocket section of the hub extension, such as the contoured section of the distal pocket.

A socket saddle can be provided with the hub body. In an example, the socket saddle can be co-molded, insert-molded, or unitarily molded with the hub body. In other examples, the socket saddle can be separately formed and subsequently attached to the catheter hub, such as be welding, adhesive, snap fit, detents, or combinations thereof. The socket saddle can include two receiving sockets, one on each side of the longitudinal axis of the catheter hub. Each receiving socket of the socket saddle can have a plurality of walls, including end walls and a bottom wall. As shown, each end wall can have a curved side edge that matches the exterior curvature of the hub body.

The bottom wall can be generally flat or planar and extends from the outer sidewall to below or underneath the hub body, wherein the bottom wall joins with the bottom wall of the second receiving socket. The unitarily formed push tab can generally align with the proximal end wall of the receiving socket but can vary. In other examples, the push tab can align with the distal end wall or somewhere between the two end walls.

In alternative embodiments, the socket saddle can have a structure with open sides or with projections so that no actual sockets are utilized to receive male projections. For example, where a tab adaptor incorporates sockets, the socket saddle can incorporate corresponding or complementary projections to project into the sockets of the tab adaptor. The socket saddle can have a width, measured orthogonally to the length of the catheter hub, that is wider than the diameter or width of the catheter hub. In an embodiment, the socket saddle can have sidewalls that are located radially, and spaced, away from the exterior of the catheter hub.

The hub extension can comprise a body comprising a tip, two wings, and a pocket or cavity for accommodating part of the catheter hub and the socket saddle. The pocket can have a distal pocket section and a proximal pocket section. The two pocket sections can have different shapes or geometries. As shown, the distal pocket section can shaped as a partial cylinder defining a cradle for accommodating the distal cylinder portion of the catheter hub. The proximal pocket section can be generally rectilinear in shape for accommodating the socket saddle, which can be subjacent part of the hub body. In other examples, the shapes of the distal socket section and the proximal socket section can differ and be complementary to the surfaces of the catheter hub.

The proximal pocket section can have a proximal wall with an arcuate cutout, a distal wall with an arcuate cutout, and two sidewalls that form part of the inner edges of the two wings. The proximal wall of the proximal pocket section can align with the two trailing edges of the two wings. In an example, the two arcuate cutouts can be aligned to accommodate a generally round cylinder. The two arcuate cutouts can have different cutout sizes to accommodate for the tapering cylinder of the catheter hub. Because the hub body of the catheter hub tapers in the distal direction, the distal cutout can have a smaller radius than the proximal cutout. The distal cutout can be modified to receive different shapes of the catheter hub.

A plurality of mating members can be provided on the bottom wall of the pocket or cavity for engaging complementary mating members at the bottom of the catheter hub, such as the bottom of the socket saddle, as further discussed below. The bottom wall can form part of the bottom surface of the hub extension. In the present embodiment, the plurality of mating members embody spaced apart projections. The projections can be short spaced apart round stubs. The short spaced apart round stubs can project into corresponding round mating members or holes on the catheter hub, or to a structure secured to the catheter hub, to secure the catheter hub to the hub extension. There can be four spaced apart stubs that align in a straight line.

In other examples, there can be fewer than four spaced apart stubs or greater than four. In still other examples, the shape of the stubs can differ, such as having oval shapes, triangular shapes, square shapes, or rectangular shapes, to name a few non-limiting examples. In yet other examples, the mating members can embody holes or openings for engaging stubs formed at the bottom of the catheter hub, or at the socket saddle attached to the catheter hub. While the mating members are shown arranged in a line along the lengthwise axis of the hub extension, the mating members can be arranged differently, such as in a line orthogonal to the lengthwise axis, near the four corners of the proximal pocket section, or randomly situated. The arrangement of the mating members on the catheter hub, such as on the socket saddle, can be positioned accordingly to engage the mating members on the hub extension.

The recessed section of the tab adaptor can be incorporated for accommodating the projection of the unitarily formed tab located at the top of the catheter hub. If the unitarily formed tab is omitted, the recessed section can also be omitted. The shape of the recessed section can be complementary to the unitarily formed tab. The shape of the recessed section can be different and/or larger than required to accommodate the unitarily formed tab.

A contoured surface can be provided in the distal pocket section, adjacent the proximal opening of the tip. The contoured surface is complementary to surfaces of the base section of the catheter hub nose section.

The perimeter profile of the two wings can be chamfered or rounded to eliminate sharp edges. The wings also have a thickness profile that varies in the radial direction, from the tip of the wing to the base of the wing. In other words, the base of each wing can be thicker than the radial outmost part of the wing. Typical prior art wings have a single thickness along the radial direction, which can cause tenting and sharp size differences between the catheter hub and the wings.

Mating members can be provided on the bottom of the socket saddle for mating with the mating members of the proximal pocket section of the hub extension. The present mating members can embody holes or openings for receiving the stubs of the proximal pocket section.

The catheter hub can assemble with a catheter tube and then assembled to the hub extension. The catheter tube can first project through the bore or lumen of the tip of the hub extension. The nose section of the catheter hub can then insert into the lumen of the tip while the hub body is lowered into the pocket of the hub extension so that the round hub body of the catheter hub settles into the curved cutout on the proximal wall and the curved cutout on the distal wall. As the hub body of the catheter hub is lowered, the complementary surfaces of the hub body and the hub extension align, and the two sets of mating members can engage to secure the two components together.

Aspects of the present invention therefore comprises a catheter unit comprising a catheter hub having a hub body, a catheter tube extending distally of the hub body, and a proximal female Luer opening. The catheter unit can further comprise a hub extension comprising a body with tip having a distal opening, a proximal opening, and a lumen extending between the two openings, a pocket or cavity located proximally of the tip, and a pair of wings extending laterally of the pocket or cavity, and wherein at least part of the hub body of the catheter hub is located in the pocket or cavity.

The catheter unit further comprises a tab adaptor which can be mounted over an upper surface of the hub body. The tab adaptor can have a lower surface that is curved or arcuate for sitting atop the hub body. The lower surface of the tab adaptor can have a recessed section for accommodating a unitarily formed push tab that is formed with the hub body.

The catheter hub can seat inside the pocket or cavity and a proximal section of the catheter hub, including the proximal end opening, and the exterior threads if incorporated, extend proximally of a proximal wall of the hub extension. The proximal wall of the hub extension can have a cutout. The bottom surface of the hub extension can be generally flat or planar. The material of the hub extension can be softer than the material of the catheter hub.

The tab adaptor can attach to the catheter hub. A socket saddle is attached to the hub body and the tab adaptor is attached to the socket saddle. The attachment can be detents, snap fit, interference fit, friction fit, or combinations thereof. The hub body of the catheter hub can have a nose section and at least part of the nose section can extend through the proximal opening of the tip of the hub extension. The socket saddle can have two receiving sockets. Each receiving socket can have a generally rectilinear shape. In other examples, each socket can have one or more round or oval sockets. Where more than one round or oval socket is utilized, the two or more round or oval sockets can be spaced from one another or can connect or communicate via a channel.

The wings of the hub extension can each have a leading edge and a trailing edge. The leading edge can slope or angled in the proximal direction. Said differently, the leading edge is not orthogonal to a lengthwise axis of the hub extension but angle to slanted in the proximal direction. Each wing can have a perimeter and wherein the perimeter is rounded and smooth, and not formed as a single thickness mold, which is typical in the prior art. In other examples, the hub extension can omit the tapered wings, as further discussed below.

An alternative hub extension is not provided with opposing wings. Instead, the body of the alternative hub extension terminates adjacent the base section of the body. The alternative hub extension has two side edges defining a maximum width of the hub extension, and wherein the width of the hub extension is less than or smaller than the width of the hub extension with the two opposing wings. However, the bottom surface still provides a generally flat planar surface that is substantially larger than the surface of a typical cylindrical hub, even without wings, and therefore provides a stable base for supporting the catheter hub from rolling compared to prior art hubs with a rounded hub body.

The generally flat surface can be seen between the distal end and the proximal end and between the two side edges. From a bottom view, the hub extension resembles a modified D-shape with a stub projection extending from the curved part of the D-shape. The modified D-shape reduces the height to width ratio of the "D". The hub extension provides a generally flat surface that spans from the nose section of the catheter hub along the length of the catheter hub to about a mid-length of the catheter hub so that the curved or rounded body of the catheter hub does not directly contact the patient.

An alternative tab adaptor can comprise an upstanding tab section attached to a horizontally positioned tab section or base. The underside surface of the base can comprise a dimple or arcuate recess for form fitting around the curvature of the catheter hub.

Spaced apart anchors may comprise spaced apart posts. There can be more than one post adjacent each of the two side edges of the base. There can be two spaced apart posts adjacent each side edge. The posts can project into receiving bores formed with the anchor saddle of the catheter hub or receiving bores formed with the hub extension. The posts can engage corresponding bores to removably engage the tab adaptor to the catheter hub or the hub extension. The posts and the complementary receiving bores can be sized and shaped to provide the requisite removable engagement for use to perform vascular access with the disclosed catheter assemblies.

In another alternative tab adaptor, the adaptor body resembles a lounge chair. The adaptor body comprises an upstanding wall section, two sidewalls, and a base section comprising raised surface features for added gripping. The base section can have a contoured bottom for form fitting around the curvature of the catheter hub. A recessed section can also be provided for accommodating the unitarily formed push tab on the catheter hub. The upstanding wall section can have a distal wall surface comprising raised surface features for added gripping.

Two spaced apart anchors can be provided at the bottom of the tab adaptor. Each of the two anchors can resemble two posts connected to one another by a web. The anchors can project into receiving bores formed with the anchor saddle of the catheter hub or receiving bores formed with the hub extension. The anchors can engage corresponding receptacles or sockets to removably engage the tab adaptor to the catheter hub or the hub extension. The anchors and the complementary receiving sockets can be sized and shaped to provide the requisite removable engagement for use to perform vascular access with the disclosed catheter assemblies.

In yet another alternative embodiment, the adaptor body comprises a concave section or dimple located on a downward slope, which slopes in the proximal direction. The proximal end most point of the dimple terminates before the end of the sloped surface, and before the proximally facing wall surface. The bottom of the tab adaptor can include a contoured bottom for form fitting around the curvature of the catheter hub and a recessed section for accommodating the unitarily formed push tab on the catheter hub.

Two spaced apart anchors can be provided at the bottom of the tab adaptor. Each of the two anchors can resemble two posts connected to one another by a web. The anchors can project into receiving bores formed with the anchor saddle of the catheter hub or receiving bores formed with the hub extension. The anchors can engage corresponding receptacles or sockets to removably engage the tab adaptor to the catheter hub or the hub extension. The anchors and the complementary receiving sockets can be sized and shaped to provide the requisite removable engagement for use to perform vascular access with the disclosed catheter assemblies.

In still yet another alternative embodiment, the tab adaptor comprises a concave or dimple located on a downward slope, which slopes in the proximal direction. The proximal end most point of the dimple terminates near the end or bottom of the sloped surface. The end most point is somewhat more angled or pointed than the curved end point of the same dimple of other embodiments.

The bottom of the tab adaptor can include a contoured bottom for fitting around the curvature of the catheter hub and a recessed section for accommodating the unitarily formed push tab on the catheter hub.

An alternative catheter unit can include a catheter hub and a unitarily formed push tab. In the present embodiment, the unitarily formed push tab can have a roughened portion and a distal portion. The roughened portion can comprise a plurality of spaced ribs or fins for improved finger grip. Alternatively, the roughened portion can embody other roughened surfaces, such as raised bumps or dimples. The distal portion can have a body that is generally rounded and tapered to form a smooth transition between the tab and the hub body of the catheter hub. The nose end or nose portion of the catheter hub can have a catheter tube projecting therefrom, which can attach to the interior of the catheter hub with a ferrule or metal bushing. A valve, a valve opener, and a needle guard or tip protector may be located inside the catheter hub, as previously discussed.

A base unit having a body with a cavity for receiving the catheter hub can be provided. The catheter hub can seat within the cavity of the base unit and can engage the base unit using detents or snap fittings. The base unit can have a length that extends from about the nose section to about the proximal end of the push tab. The base unit can have a flat base bottom. The flat base bottom can provide a flat surface with the patient's skin to minimize rotation or movement of the catheter hub during connector change out. The base unit can have sidewalls that can be flat and vertical, but can alternatively be arcuate, flared, or tapered. In still other examples, the catheter hub and the base unit may be unitarily formed. When separately formed, the catheter hub and the base unit may be made with similar materials as the catheter hubs and hub extensions discussed elsewhere herein. In some embodiments, the catheter unit, including the base unit, can seat within a cavity or pocket of a hub extension so that the flat bottom surface of the hub extension seats against the patient.

In yet another embodiment of a catheter unit, the base unit has a length that is relatively shorter. The distal end of the base unit can be recessed from the nose section of the catheter hub. The distal end can be located at about the half-way mark of the hub body, below the distal portion of the push tab and can extend to approximately the roughened portion of the push tab.

In yet another example, the push tab has a structure with a sloped proximal portion and a sloped distal portion. The two sloped portions meet along a crest. The two sloped portions can both have roughened surfaces, such as having bumps or projections.

In another alternative catheter assembly, the sloped distal portion of the push tab is shortened and has a relatively steep slope. The two sloped portions meet along a crest, which is more pointed than elongated compared to the crest of other embodiments. The two sloped portions can both have roughened surfaces, such as having bumps or projections. In other examples, the base unit can extend longer from just proximal of the end of the nose section to about the proximal sloped portion.

In alternative embodiments, the push tabs of the catheter units may be separately formed from the catheter hubs and removably attachable therewith, such as with detents or snap fit.

In yet another alternative embodiment, the catheter unit has removable sub-components. For example, the catheter hub can removably engage with the base unit and the tab adaptor can removably engage with the catheter hub. In some examples, the tab adaptor is resilient and flexible and can be non-removable from the catheter hub.

The base unit can be a longer base unit or has a body that is relatively longer. The catheter hub can resemble a standard catheter hub, one without integrated wings. However, the hub can be modified to engage the base unit, such as by detents, snap fit, interference fit, adhesive, or by welding.

The tab adaptor can have a body that resembles a modified pyramid, with multiple sides and a ridge. The various sides can be generally flat or planar and sloped. The slopes of the different sides can be the same or different. In the example shown, the slope of the proximal side is less than the slope of the distal side. A plurality of bumps or projections can be incorporated on the surface of the proximal side for improved gripping. An arcuate lower surface can be provided for fitting over the exterior of the catheter hub. The tab adaptor can removably engage with the catheter hub, such as with detents, interference fit or snap fit. Although not shown, a recessed section can be incorporated to accommodate the unitarily formed push tab formed with the catheter hub.

In another example, the tab adaptor can have a flat or rounded bottom surface, but without the need for a recessed cavity since the catheter hub does not incorporate any push tab. The spaced apart posts on the tab adaptor can engage the spaced apart receiving sockets. The receiving sockets can have contoured surfaces for mating engagement with contoured surfaces of the anchors, for removable engagement between the two.

In yet another example, the two sockets on each side of the socket saddle can be generally round and a narrow channel connects the two sockets. The two sockets and the channel can be sized and shaped to engage corresponding anchors on a tab adaptor. The catheter hub can engage the base unit using detents, press fit, or interference fit. Alternatively, the base unit can be glued, bonded, or welded to the catheter hub. In still other examples, the base unit can be molded with the catheter hub, such as over-molding. The catheter hub and the base unit can optionally engage a hub extension so that the hub extension, with wings, can rest against the patient.

In yet another example, the hub extension comprises a body comprising a pair of wings extending laterally of a pocket or cavity. Two mounds can be provided atop the wings. The two mounds define the pocket or cavity. Each mound can comprise a block comprising a plurality of wall surfaces. The wall surfaces can be generally flat and can be angled to one another. Two angled or ramped surfaces can be incorporated. Each of the side angled or ramped surface on the two wings can gradually decrease the height profile of the mount so that when adhesive dressing is applied, tenting is reduced. Similarly, each of the front angled or ramped surface on the two wings gradually decreases the height profile of the mount at the front so that when adhesive dressing is applied, tenting is reduced.

In an example, a distal wall on a hub extension is provided with a cutout 280 accommodating the generally round profile of the catheter hub. In an example, the cut-out includes an upper rounded portion and a lower straight section. In other examples, the cut-outs can have different shapes. The shapes can depend on the shape of the catheter hub and/or base unit. Said differently, the cut-outs can be sized and shape to matingly receive the catheter hub and/or the base unit.

In an example, the proximal end of the cavity or pocket is open, or not obstructed. The open configuration is configured to receive a generally wide and flat base unit. As shown, the cavity sidewalls terminate at respective proximal ends without any other wall or structure to provide for the open configuration. In other examples, the proximal end of the pocket or cavity is confined by a proximal wall with a cutout. The cutout is sized and shaped to accommodate the catheter hub and/or the base unit.

Any of the catheter units described herein are understood to include a catheter tube, a needle, a needle hub, and optionally one or more of a needle guard, a valve, a valve opener, and a third housing for housing the needle guard, as described elsewhere herein.

Methods of making catheter assemblies and catheter units and components thereof as described elsewhere herein are within the scope of the present invention.

### BRIEF DESCRIPTION OF THE DRAWINGS

These and other features and advantages of the present devices, systems, and method will become appreciated as the same becomes better understood with reference to the specification, claims and appended drawings wherein:
FIG. 1 is a cross-sectional side view of a catheter assembly comprising a catheter hub and a needle hub and wherein a needle guard is located inside the catheter hub.
FIG. 2 shows a side view and partial cross-sectional view of an alternative catheter assembly in accordance with aspects of the invention.
FIGs. 3A, 3B, and 3C are different views of the same catheter unit, which includes a catheter hub with a catheter tube (not shown), a hub extension, and tab adaptor.
FIG. 4A is an exploded view of a catheter unit, showing a tab adaptor separated from the catheter hub.
FIGs. 4B and 4C schematic views of exemplary interfaces between the anchors of the tab adaptor and the receiving sockets.
FIGs. 5A, 5B, and 5C are exploded perspective views of components of a catheter unit.
FIGs. 6A and 6B are additional views of the hub extension and catheter hub of FIGs. 5A and 5B.
FIGs. 7A, 7B, and 7C are different views of the same catheter unit without wings or with limited wings.
FIG. 8 is an exploded view of a catheter unit, showing a tab adaptor separated from the catheter hub.
FIGs. 9A and 9B are perspective views of the hub extension and catheter hub of FIG. 8, separated from one another.
FIGs. 10A and 10B are perspective views of an alternative tab adaptor provided in accordance with further aspects of the invention.
FIGs. 11A and 11B are perspective views of another alternative tab adaptor provided in accordance with further aspects of the invention.
FIGs. 12A, 12B, and 12C are perspective views of yet another alternative tab adaptor provided in accordance with further aspects of the invention.
FIGs. 13 and 14 are perspective views of still yet another alternative tab adaptor provided in accordance with further aspects of the invention.
FIGs. 15 and 16 are perspective views of alternative catheter units provided in accordance with further aspects of the invention, one with a long base and one with a relatively shorter base.
FIGs. 17 and 18 are perspective views of additional alternative catheter units provided in accordance with further aspects of the invention, one with a long base and one with a relatively shorter base.
FIG. 19 is a perspective view of yet another alternative catheter unit provided in accordance with further aspects of the invention.
FIG. 20A is a perspective view of yet another alternative catheter unit provided in accordance with further aspects of the invention and FIG. 20B is a perspective view of the tab adaptor separated from the catheter hub.
FIG. 21A is a perspective view of yet another alternative catheter unit provided in accordance with further aspects of the invention in which the catheter hub does not incorporate a push tab and FIG. 21B is a perspective view of a tab adaptor usable with the base member thereof.
FIG. 22 is a perspective view of yet another alternative catheter unit provided in accordance with further aspects of the invention in which the catheter hub has an integrated tab and usable with a base unit.
FIGs. 23 and 24 are perspective views of alternative hub extensions provided in accordance with further aspects of the invention.
FIGs 25 and 26 are catheter units provided in accordance with still further aspects of the invention.

### DETAILED DESCRIPTION

The detailed description set forth below in connection with the appended drawings is intended as a description of the presently preferred embodiments of needle devices, such as catheter assemblies and peripheral type catheter assemblies, provided in accordance with aspects of the present devices, systems, and method and is not intended to represent the only forms in which the present devices, systems, and methods may be constructed or utilized. The description sets forth the features and the steps for constructing and using the embodiments of the present devices, systems, and method in connection with the illustrated embodiments. It is to be understood, however, that the same functions and structures may be accomplished by different embodiments that are also intended to be encompassed within the scope of the present disclosure. As denoted elsewhere herein, like element numbers are intended to indicate like or similar elements or features.

Descriptions of technical features or aspects of an exemplary configuration of the disclosure should typically be considered as available and applicable to other similar features or aspects in another exemplary configuration of the disclosure. Accordingly, technical features described herein according to one exemplary configuration of the disclosure may be applicable to other exemplary configurations of the disclosure, and thus duplicative descriptions may be omitted herein.

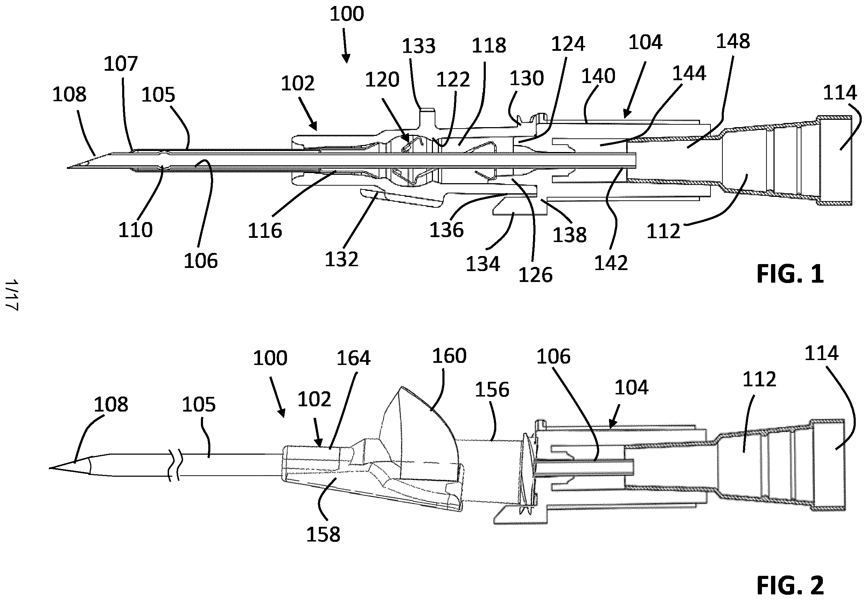

With reference now to FIG. 1, a cross-sectional side view of a needle assembly or needle device 100 provided in accordance with aspects of the present disclosure is shown, which comprises a first hub 102 having an over-the-needle tube or tubing 105 attached thereto and a second hub 104 having a needle 106 attached thereto. The tubing 105 has a lumen or bore for receiving the needle and has a distal opening or distal end opening 107. The needle assembly 100 can be called a catheter assembly or a catheter device. The needle device or assembly 100 is configured for peripheral access but components used therein can apply to other or different applications. The needle 106 has a needle tip 108 and can include a change in profile 110 located proximal of the tip 108 for use with a needle guard. The needle tip 108 extends distally of the distal end opening 107 of the tube or tubing 105 in the ready to use position with the change in profile 110, if incorporated for use with a needle guard, located proximally of the distal tube opening. The change in profile 110 can be a crimp, a bump, or a material build-up having different profile than other diameter sections of the needle shaft. As used herein, the term proximal is understood to mean an end or side closer to the practitioner and the term distal is the opposite end or side.

The change in profile 110 can be used to interact with a needle guard during retraction of the needle, as further discussed below. The first hub 102 may alternatively be referred to as a catheter hub and the second hub 104 may alternatively be referred to as a needle hub. A vent plug 112 is disposed at the proximal open end 148 of the second hub 104 and has a vent filter 114 at a proximal end thereof, which is conventional. As shown, the tube 105 is attached to the first hub 102 by a ferrule or metal bushing 116 and may be referred to as a catheter tube.

A needle guard 120 can optionally be provided in the interior cavity 118 of the first hub 102 for covering the needle tip 108 in the protective position, such as following successful venipuncture. When incorporated, the needle guard 120 can be one of the needle guards disclosed in US Patent No. 6,616,630. In some examples, the needle guard 120 can be omitted. If so, the change in profile can also be omitted. In still other examples, a needle guard can be included without also including a change in profile on the needle. For example, the needle guard can have a perimeter defining an opening and the opening can cant or lean over when activated to grip the needle. In yet other examples, the needle guard 120 can be located in a third housing between the first hub and the second hub. An exemplary needle guard located in a third housing is disclosed in US Pat. No. 8,597,249.

The needle guard 120 can include a proximal wall and two arms extending distally of the proximal wall. The two arms can intersect one another in the ready to use position of FIG.1 and in the protective position in which the needle guard covers the needle tip, and as viewed from a side. In some examples, the two arms of the needle guard 120 can extend in a distal direction without intersecting one another. Two distal walls, one on each arm, can be incorporated to block the needle tip. The two distal walls are biased outwardly by the needle 106 in the ready to use position of FIG. 1 and disposed distally of an internal change in profile 122 inside the first hub 102 in the ready to use position. The internal change in profile 122 can be a reduced diameter section located next to an inside diameter section of a larger dimension. Each distal wall can include a curved lip to facilitate relative movement between the needle guard and the needle. The two distal walls can also each have a curved lip at an end thereof to improve sliding movement along the needle shaft.

When in the ready to use position, the dimension measured between the two joints or elbows at each arm, at the intersection between each arm and its respective distal wall, is larger than the inside diameter of the internal change in profile 122, which prevents the needle guard 120 from moving proximally thereof. Once the needle tip moves proximally of the two distal walls and the arms no longer biased by the needle shaft, the two arms move radially inward and one or both distal walls close over the needle tip to block the needle tip in a protective position. At that point, the dimension between the two joints or elbows decreases and is smaller than the internal dimension of the internal change in profile 122, which then allows the needle guard to move proximally and be removed from the catheter hub with the needle.

The first hub 102 has a proximal opening 124 having a nose section 126 of the second hub 104 disposed therein. The proximal opening 124 has a female Luer for receiving a male Luer tip, such as a syringe, an IV tubing adaptor, a Luer extension, etc. External threads 130 can be provided on the exterior surface at the proximal end for threaded engagement with a threaded collar of a male Luer tip. A pair of stabilizing wings 132 can extend radially of the first hub to facilitate securement or anchoring of the first hub 102 to a patient following successful venipuncture. Optionally the wings can be omitted. The first hub 102 can embody a standard IV catheter hub without a tubing port. In other examples, the first hub can include a tubing port and a tubing connected thereto, with a needleless valve connector connected on the opposite end of the tubing, which is also known as an integrated catheter. In still other examples, the first hub 102 can include a side fluid port, also known as a ported catheter. Externally, a push tab 133 can be unitarily formed with the hub body. The push tab 133 can be used by a practitioner to grip and advance the needle device 100, such as to gain peripheral access.

The first hub 102 is removably secured to the second hub 104 by receiving the nose section 126 of the second hub in the proximal opening 124 of the first hub. A flange or extension 134 is provided on the second hub 104 and together with the nose section 126 define a gap 136 having part of the first hub located therein. Optionally the flange or extension 134 can be omitted and a stub 138 or other surface mating features provided. The stub 138 can limit the extent of insertion of the second hub 104 into the first hub 102. A rib or annular flange can also extend from the nose section 126 of the second hub 104 to limit the extent of insertion of the nose section into the first hub.

The second hub 104 has a body 140 having an interior cavity 144 having the proximal end 142 of the needle 106 projecting there-into. The interior cavity 144 can function as a primary blood flashback chamber. The proximal opening 148 of the body 140 can have a female Luer for receiving a vent plug 112 therein and the exterior can have threads or be without threads. In an example, the exterior of the body 140 of the second hub 104 is generally cylindrical and without threads for receiving an adjustable grip or extension clip, as further discussed below. The first and second hubs can be made from plastic materials, such as by plastic injection.

With reference now to FIG. 2, a side view of an alternative needle assembly or needle device 100 provided in accordance with further aspects of the invention is shown, which is similar to the needle device of FIG. 1 with several changes. The present embodiment comprises a catheter hub 102 having a catheter tube 105 extending therefrom and a needle hub 104 with a needle 106 projecting through the catheter hub and the catheter tube and having a needle tip 108 extending out a distal end of the catheter tube. A vent filter 114 is connected to the needle hub and can be similar to that of FIG. 1. Internally of the catheter hub 102, the assembly may optionally include a needle guard like that of FIG. 1. In still yet other examples, the catheter hub 102 may further include a valve and a valve opener located in the interior of the catheter hub. The valve can comprise a plurality of slits defining a plurality of flaps and the valve opener can comprise a nose section and one or more actuating elements extending in the proximal direction from the nose section. The valve opener can be pushed in a distal direction by a male Luer tip, such as a syringe tip, to open the valve flaps. Catheter assemblies with a needle guard, a valve, and a valve opener that are usable with catheter assemblies of the present application are disclosed in U.S. Publication No. 2018/0214682 A1 to Woehr et al. and in U.S. Publication No. 2020/0188634 A1 to Woehr et al..

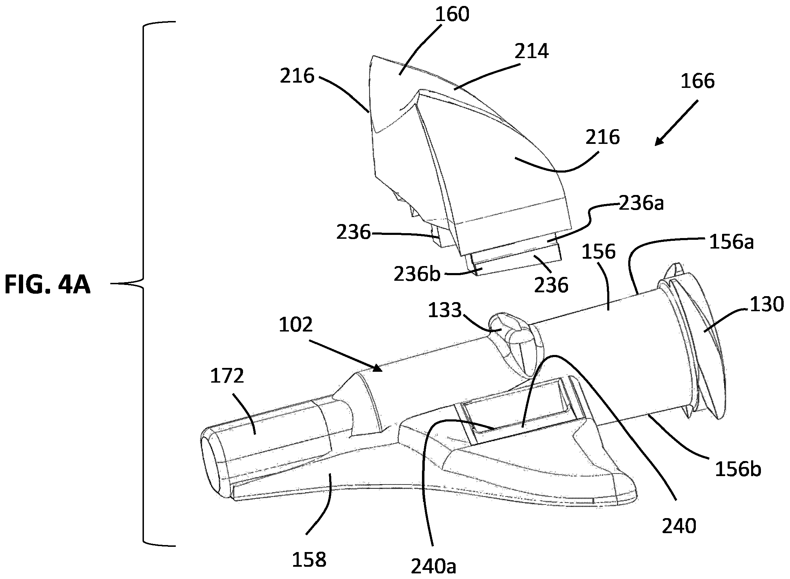

As shown, the present needle device 100 comprises a catheter hub body 156 having an interior, which can accommodate at least one of a needle guard, a valve, and a valve opener. The catheter tube 105 can attach to the distal end of the hub body 156 with a metal bushing or ferrule, which is standard or traditional. The hub body 156 further comprising an exterior having an upper surface 156a located above, elevation-wise, a lower surface 156b (FIG. 4A). Also shown and further discussed below is a hub extension 158 and a push tab adaptor 160. The hub extension 158 can include a body 164 having a receiving space that is sized and shaped to receive the catheter hub body 156. The hub body 156 typically has a body that is rounded or not substantially flat, which can shift or rotate during use. The hub extension 158 provides an enlarged surface area so that when the hub extension 158 is secured to the patient following successful venipuncture, the enlarged surface area provides comfort and stability from rotating, such as during connector change.

In an example, the push tab adaptor 160 is removably attached to the hub body 156, such as by interference, tapered fit, friction fit, snap fit, detents, or equivalent attachment means or connection means. In other examples, the tab adaptor 160 is removably attached to the hub extension 158. A typical unitarily formed push tab has a first size that is configured for pushing or manipulating when using the catheter assembly. The push tab adaptor 160 of the present embodiment provides enlarged gripping surfaces above and beyond the first size, i.e., enlarged to a second larger size, for a practitioner to grip and push, and/or pull, the needle assembly when performing vascular access. The enlarged gripping surfaces provides stability, improved access to gripping, and less prone to rotation during use. As further discussed below, the push tab adaptor 160 can engage the hub body 156 or alternatively can engage the hub extension 158 for use during vascular access so that a relatively larger surface is made available for the user to use during insertion of the needle and catheter. The enlarged push tab adaptor provides a large surface for the user to grip and manipulate and as well as shield the catheter hub body 156 from direct contact by the user by serving as the intermediary. The push tab adaptor 160 can subsequently be removed following successful venipuncture. The enlarged surface can increase the width, the height, or both the width and the height of the unitarily formed push tab.

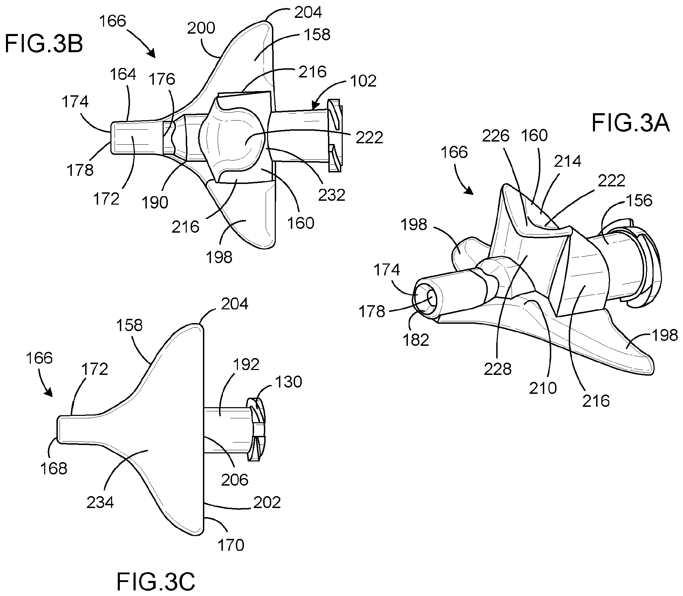

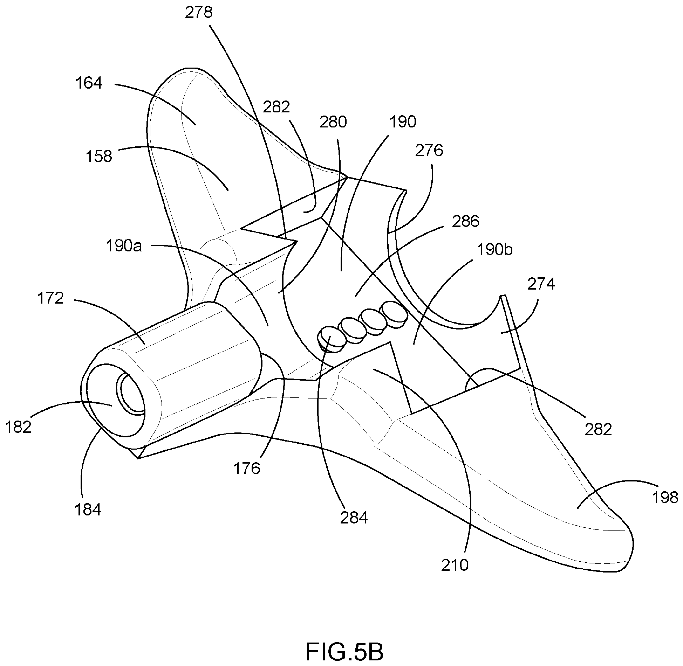

The combination catheter hub 102, hub extension 158 and tab adaptor 160 is shown in FIGs. 3A, 3B, and 3C, which show a front perspective view, top view, and bottom view, respectively, of the device. The combination catheter hub 102, hub extension 158, tab adaptor 160, and catheter tube 105 (FIGs. 1 and 2) attached to the catheter hub 102 may herein be referred to as a catheter unit 166. For simplicity, the catheter tube 105 has been omitted from the drawings but is understood to be part of the catheter unit 166. Further for simplicity, a needle hub 104 and needle 106 for use with the catheter unit 166, as shown in FIG. 2, have been omitted from the drawings but are understood to be usable with the catheter unit 166. As shown, the hub extension 158 has a body 164 comprising a first end or distal end 168 and a second end or proximal end 170, which define a length therebetween. In an example, the hub extension 158 has a tip 172 comprising a length (FIGs. 3A and 3B) that is shorter than the length of the hub extension 158. The tip 172 has a distal end with a distal opening 174 and a proximal end with a proximal opening 176 and a lumen 178 extending between the two openings. As further shown with reference to FIG. 5B, the distal end 174 of the tip 172 has a ramped surface 182. The ramped surface 182 resembles truncated frustoconical structure, such as a truncated cone structure, with a relatively thin leading edge 184. This ramped surface 182 allows the catheter tubing 105 (FIGs 1 and 2) to form a controlled bend, or provides a surface that supports the catheter tube, when in use so as to avoid kinking.

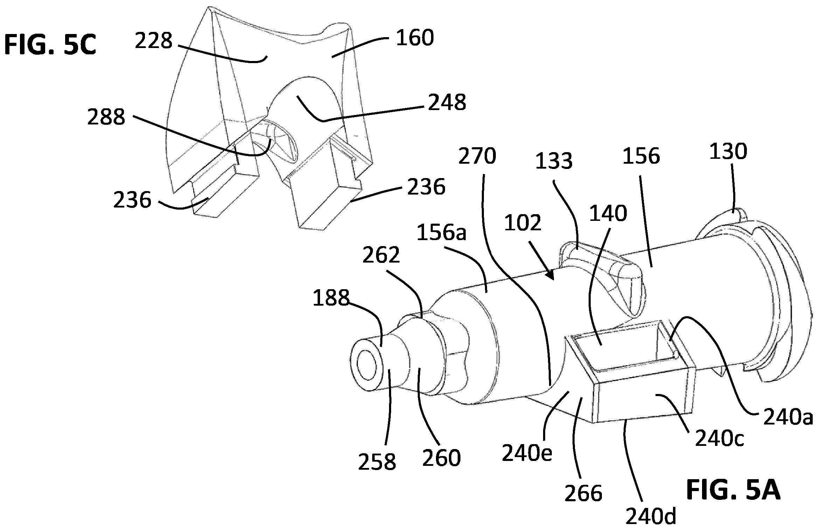

The proximal opening 176 of the tip 172 is sized and shaped to receive the nose section 188 (FIG. 5A) of the catheter hub 102. In an example, the proximal opening 176 has corresponding negative surfaces or complementary surfaces as the nose section 188 for receiving the nose section 188 of the catheter hub. The bore or lumen 178 of the tip 172 is sized and shaped to accommodate the catheter tube. Thus, part of the nose section 188 and catheter tube 105 are both located in the lumen 178 of the tip 172. The clearance or tolerance between the lumen 178 and the catheter tube should be a loose fit or a size-on-size fit. The tip 172 can have a generally cylindrical exterior. As shown, the tip 172 has a generally polygonal exterior with chamfered or rounded corners connecting the sides. The exterior surfaces of the tip can be smooth or can be roughened, such as having textures, or both.

The body 164 of the hub extension 158 includes a cavity or pocket 190 (FIG. 5B) for engaging or receiving part of the hub body 156 of the catheter hub 102, with part of the proximal cylinder 192, including the Luer threads 130, extending proximally of the second end 170. In some examples, the catheter hub 102 can incorporate a pocket and the hub extension 158 can include a projection that projects into the pocket of the catheter hub to secure the two components. A pair of wings 198, 198 extend laterally of the length of the body 164 of the hub extension, or laterally of the hub body 156. Each of the two wings 198, 198 has a leading edge 200, a trailing edge 202 and an apex or connecting portion 204 that connects the two edges. In an example, the leading edge 200 is curved or arcuate while the trailing edge 202 is generally straight. Starting from about the proximal end of the tip 172, each of the two wings 198, 198 angled in the proximal direction (FIG. 3C) and curves as it extends in the proximal direction. As shown in FIG. 3C, an embodiment of the present invention includes trailing edges 202 for the two wings 198, 198 that generally align, such as in a generally straight line. In a particular example, the two trailing edges 202 of the two wings form a generally straight line or edge with a proximal edge 206 of the central body portion. From a bottom view (FIG. 3C), the hub extension 158 resembles a D-shape, along a top view or bottom view, with a stub projection extending from the curved part of the D-shape. The hub extension 158 provides a generally flat surface that spans from the nose section of the catheter hub along the length of the catheter hub to about a mid-length of the catheter hub so that the curved or rounded body of the catheter hub does not directly contact the patient. In alternative embodiments, the trailing edges 202, and possibly the proximal edge 206 of the central body portion, can include some non-linear or curved sections. For example, the entire proximal edge of the body 164 can bow outwardly in the proximal direction to further increase the size of the bottom surface 234.

With specific reference to FIG. 3A, each wing 198 has a base portion 210 having a curved or sloped surface. In the catheter unit 166 assembled configuration shown, the generally cylindrical hub portion of the catheter hub body 156 and the sloped base portion 210 have a dividing or split line therebetween, formed by snapping the catheter hub 102 into the pocket or cavity 190 of the hub extension 158, as further discussed below. The two base portions 210 of the two wings and the upper portion of the catheter hub body 156 form a generally continuous smooth hump, with some change in inflection possible. Said differently, the curvature of the catheter hub body 156 and the sloped base portion 210 form a smooth transition that gradually decreases the height of the catheter unit 166 as it extends towards the tip or apex 204 of each wing 198. Thus, when the tab adaptor is removed following vascular access, as further discussed below, the remaining structure has a smooth contour that facilitates securement of the adhesive dressing.

The tab adaptor 160 is located over or on top of the catheter hub body 156. The tab adaptor 160 can be removably attached to the catheter hub body 156, as further discussed below. In other examples, the tab adaptor is removably attach to the hub extension 158, such as via detents, snap fit, interference fit or combinations thereof. In an embodiment, the tab adaptor 160 has a body 214 with a width and wherein the width of the body 214 is wider than the width of the cylindrical catheter hub body 156. In an example, the width of the catheter hub is X, and the width of the tab adaptor is 1.1X to 2.5X, and more preferably from 1.5X to 2.2X the width of the catheter hub. The enlarged width and height of the tab adaptor provide stability and a large surface to grab, grip, or contact by the user so as to minimize directly contacting the catheter hub body and presents a stable surface for use during vascular access. As further discussed below, the body 214 of the tab adaptor 160 has two opposed sidewalls 216, 216 and a concave surface or dimple 222 located between the two sidewalls and presenting a contoured surface for pushing or pulling. The two sidewalls 216, 216 can each have the shape of a sail or shark dorsal fin. The curved part of each of the two sidewalls can orient in the proximal direction while the more vertical or straight part of the sidewall faces the distal direction. Thus, each sidewall has a straight part, a curved part, and a connecting part connecting the straight part and the curved part.

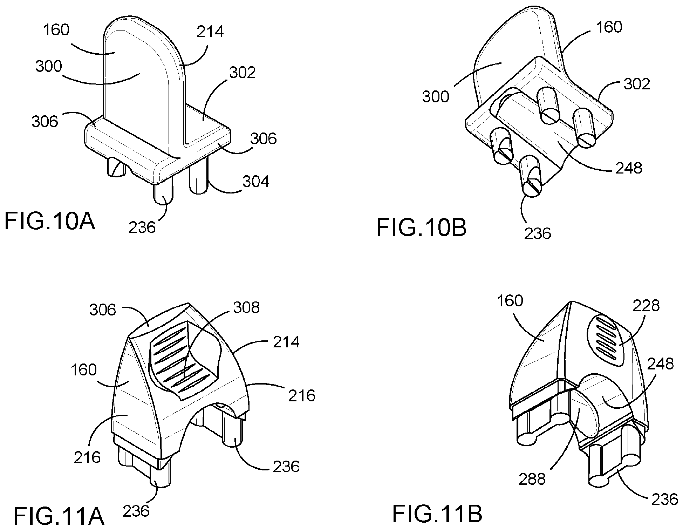

The concave surface 222 provides a cradle or seat for the finger or fingers for improving finger grip when using the tab adaptor 160 to advance the catheter assembly. In some examples, textured features, such as bumps, small recessed dimples or fins, maybe included on surfaces of the tab adaptor, such as the concave surface 222, to provide added gripping features. Exemplary textures are shown on the base section 308 of FIG. 11A. The concave surface 222 can also slope downward in the proximal direction. The sloping surface allows the user to push the tab 160 in the distal direction with better leverage during vascular access. Alternatively, the user can use the crest 226 at the upper distal end of the concave surface 222 and the distal wall 228 of the tab adaptor to wedge a finger against and pull or push the catheter assembly, as the case may be, when performing vascular access. In some examples, the concave surface 222 can extend proximally but not sloped as it extends proximally. In alternative embodiments, the surface between the two sidewalls 216 can sloped downwardly in the proximal direction but can be flat or planar as it slopes.

A crest or ridge 226 is formed at the intersection of the concave surface 222 and the distal wall 228, between the two sidewalls 216, 216. In an example, the crest 226 is arc shape. The center of the crest 226 is below, elevation-wise, the highest points on the two sidewalls 216, 216. Thus, a finger placed at the crest 226 is cradled by the surrounding surfaces to provide stability and grip. The distal wall 228 is convex and bulges distally, as shown in FIG. 3B. In some examples, the distal wall 228 can be flat or planar or even concave. In an example, the proximal wall 232 of the tab adaptor 160 is generally flat or planar. The proximal wall 232 can align with the trailing edge of the hub extension 158. Optionally, the proximal wall 232 can bulge proximally to present additional surfaces for gripping.

In an example, the hub extension 158 can be made from a soft thermoplastic material or a soft thermoplastic elastomer (TPE) material, having a softer feel than the material used to form the catheter hub 102. Preferably, the soft thermoplastic material or the soft TPE material is soft and flexible. Preferably, the soft thermoplastic material or the soft TPE material is flexible and at least the bottom surface 234 (FIG. 3C) of the hub extension 158 is smooth for improved patient comfort. That is, the bottom surface 234 can be generally flat and the flat surface can extend from the tip to the proximal end 170, such as to the trailing edges of the wings. The wide and general flat bottom surface 234 provide a stable base to prevent rotation of the catheter hub, such as during use, during patient movement, and during connector change out, which in turn improves patient comfort. The enlarged and general flat bottom surface 234 distribute forces from being concentrated at any uneven point on the catheter unit that may rise or extend above other points or surfaces on the hub extension. With this, the pressure is equally distributed to prevent discomfort, pressure ulcers and pressure markings. In some examples, one or more recessed channels can be provided at the bottom surface 234 to provide ventilations.

The soft material used to form the hub extension 158 is pliable and can partially conform to the patient for added patient comfort. In an example, the catheter hub 102 can be made from a polyurethane material. The catheter hub 102 may alternatively be made from a polypropylene (PP) material with other conventional materials for forming catheter hub contemplated. The hub extension 158 can be made from low density polyethylene (LDPE), polyethylene terephthalate (PET), or polyvinyl chloride (PVC), or equivalents. Alternatively, the hub extension 158 can be made from a TPE material, such as thermoplastic olefins, styrene SBS (styrene-butadiene-styrene block copolymer), SEBS (styrene-ethylene-butylene-styrene block copolymer) or SEPS (styrene-ethylene-propylene-styrene block copolymer) compound, vulcanized PP/EPDM (ethylene propylene diene monomer rubber) compound, thermoplastic polyurethane, or thermoplastic polyamide. Other non-limiting examples include polycarbonate (PC), glass filled nylon, PP, high density polyethylene (HDPE), polystyrene, and acrylonitrile butadiene styrene (ABS).

The catheter hub 102 has a lengthwise axis extending from the distal end to the proximal end of the hub body 156. A standard prior art catheter hub, when placed on a flat planar surface, typically has a lengthwise axis that is approximately parallel to the flat surface. In the present embodiment, the lengthwise axis of the catheter hub is angled to the flat surface, such as angled to the ground or to the flat bottom surface 234 of the hub extension 158, by about 1 degree to about 15 degrees with 3 to 8 degrees being more preferred. The angled configuration can be arranged by adjusting how the catheter hub 102 is positioned relative to the hub extension 158 and the hub extension 158 suspending the catheter hub in a way that the lengthwise axis is angled relative to the flat surface. The angled configuration allows the distal tip of the hub extension 158 to be angled closer to a surface, such as to a patient's skin, to minimize or eliminate potential catheter tube kinking.

With reference now to FIG. 4A, a partially exploded perspective view of the catheter unit 166 of FIGs. 2 and 3A-3C is shown with the tab adaptor 160 separated or spaced from the catheter hub 102. The separated position shown is analogous to when vascular access or successful venipuncture has been achieved and the tab adaptor 160 is separated from the catheter hub 102 before the catheter hub is then secured to the patient, such as by adhesive or medical dressing. As shown, the catheter hub 102 has a unitarily formed push tab 133. The push tab 133 has a height and a width, which can be standard or conventional. In an example, the height of the push tab 133 of the present invention can be shorter or smaller than traditional push tabs formed with traditional catheter hubs. The push tab 133 enables the practitioner to adjust or manipulate the catheter hub should the tab adaptor 160 be removed. When a relatively shorter and/or smaller push tab 133 is incorporated, the issue of tenting is reduced. Tenting arises when a tape, adhesive or dressing is placed over the catheter hub and the projection of the push tab 133, rising above the exterior surface of the hub body 156, creates un-even and non-contacting surfaces with the adhesive, which in turn traps air pockets and reduces effectiveness of the dressing adhesion. In some examples, the unitarily formed push tab 133 can be omitted from the catheter hub as the removable tab adaptor 160 can serve as a structure for pushing or manipulating the catheter hub without also utilizing the unitarily formed push tab.

The tab adaptor 160 has two anchors 236, 236 extending downwardly from the body 214. The two anchors 236, 236 can be spaced from one another. Each of the two anchors 236, 236 can locate below, elevation-wise, the lower surface of the tab adaptor and generally aligns with a corresponding sidewall 216, 216. In some examples, each anchor can recess from a side edge or sidewall of the tab adaptor. The two anchors can be substantially similar or identical. Each anchor can comprise a height and a width. Each anchor can comprise an extended surface 236a and a radial surface 236b. The two surfaces 236a, 236b of the anchor can resemble a barb or a male detent. In other examples, each anchor resembles a stub. The far end of each anchor, remote from the bottom of the tab adaptor, can have a greater thickness, measure orthogonally to the extended surface 236a, than near the upper end adjacent the bottom of the tab adaptor. This arrangement minimizes potential for trapping air when the anchors are connected to the hub body. In other examples, gaps or channels between surfaces of the anchors and the receiving sockets of the catheter hub can be incorporated to minimize air trap.

The catheter hub 102 of the present embodiment comprises a receiving socket 240 located laterally of the lengthwise axis of the catheter hub. Preferably, a receiving socket 240 is provided on either side of the lengthwise axis of the catheter hub. Each socket 240 can be sized and shaped to receive a corresponding anchor 236 extending at the bottom of the tab adaptor. In some examples, the receiving socket can have a bottom that is deeper than the reach of the anchor 236. This extra space can be provided to incorporate a closure system for closing the opening of the receiving socket when the anchor is removed, as further discussed below.

In an example, each socket 240 has a perimeter 240a defining an opening. The perimeter 240a can have multiple sides, or have a polygonal configuration. In other examples, the perimeter can have curved edges and can have an irregular shape. The perimeter 240a and the anchor 236 can have corresponding shapes so that the anchor 236 can project into the receiving socket 240. As shown, the perimeter 240a of the socket defines a plane and wherein the plane of the receiving socket 240 is slanted or angled relative to a horizontal plane, which can be the ground, the patient's skin, or the plane defined by the bottom surface 234 of the hub extension 158 (FIG. 3C). The slant can be downward as the opening extends radially of the lengthwise axis. In other examples, the plane can be horizontal or even slants upwardly as it extends radially of the lengthwise axis. As shown, the downward slant of the opening 240a generally coincides, matches, or aligns with the slanted or curved profile of the base portion 210 of the wing 198. This shape or configuration provides a smooth transition between the catheter hub 102 and the hub extension 158 when the tab adaptor 160 is removed, which improves bonding with the adhesive dressing and reduces the number and size of potential air pockets.

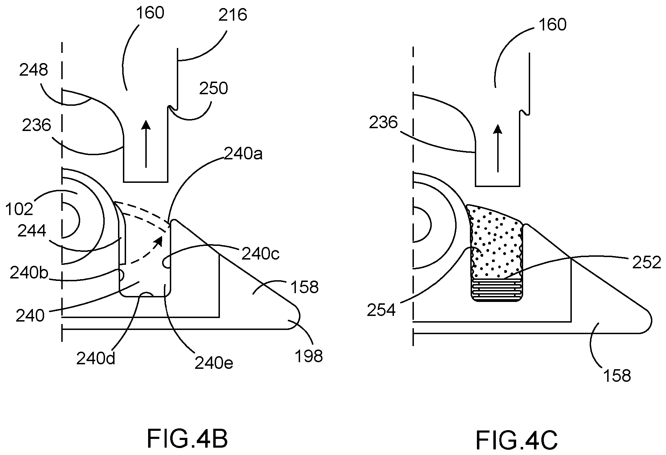

FIG. 4B is a schematic partial cross-sectional end view of the separated catheter unit 166 of FIG. 4A with an alternative anchor 236 extending at the bottom of the tab adaptor 160 and the receiving socket 240 of the catheter hub 102. As shown, the receiving socket 240 has an upper opening 240a, two sidewalls 240b, 240c, and a bottom wall 240d. The receiving socket 240 also has two end walls 240e, 240e at two ends of the sidewalls. The various walls define a socket for receiving the anchor 236. The various walls can be generally orthogonal to one another and intersections between the walls can be radiused. The upper opening 240a and the bottom wall 240d define a receiving socket depth, which can be variable because of the angled upper opening. The receiving socket depth can have the same but negative or complementary size as the anchor. Alternatively, the receiving socket depth can be deeper than the length of the anchor. The size difference can serve as space for incorporating additional features, such as a foam, biasing member, or helical compression spring to aid in pushing the anchor upwardly to assist with removal of the tab adaptor and/or to deploy a cover for covering the upper opening 240a following removal of the tab adaptor. In an example, a pliable sleeve, such as a thin rubber sleeve is provided over a compression spring and the combination is located in the receiving socket 240. As the anchor 236 is inserted into the receiving socket, the anchor compresses the spring and the sleeve. When the anchor is removed, the spring expands and pushes the sleeve upward to cover the upper opening 240a of the receiving socket 240.

As shown in FIG. 4B, a rotatable or bendable flap 244 or a flap that swings is provided at the upper opening 240a of the receiving socket 240. The flap 244 can be unitarily formed with the body 164 of the hub extension 158, or can be attached to the body 164, such as by adhesive or by welding. When incorporated, the size of the receiving socket 240 can be adjusted accordingly to accommodate both the flap 244 and the anchor 236. The flap can swing inwardly into the interior space of the receiving socket 240 when the anchor is inserted and can swing upwardly to at least partially cover the opening 240a when the anchor is removed.

As shown, the tab adaptor 160 has a lower surface 248 that is curved. The lower surface 248 and the curvature of the hub body 156 of the catheter hub 102 are complementary so that when the tab adaptor 160 is removably secured to the catheter hub 102, the two surfaces are form-fitting. In some examples, the anchor 236 and the sidewall 216 of the tab adaptor have coplanar surfaces. However, the anchor 236 can recess from the sidewall 216 and a lip 250 can extend radially of the anchor. The lip 250 can abut or contact the perimeter of the upper opening 240a or the wing when the tab adaptor is secured to the hub.

FIG. 4C is a schematic cross-sectional end view of the separated catheter unit 166 of FIG. 4A with an alternative receiving socket 240 on the catheter hub 102. The current receiving socket 240 is similar to the receiving socket 240 of FIG. 4B but wherein a pliable or biasing unit 252 is located in the interior of the receiving socket 240. The biasing unit 252 can be a combination of a compression spring and a sleeve, can alternatively be a spring and a telescoping structure, or can alternatively be a porous foam or sponge material. The biasing unit 252 can compress upon being pushed by the anchor and can expand upon removal of the anchor to cover at least part of the upper opening 240a of the receiving socket 240.

In an example, the walls of the receiving socket 240 can be provided with gripping features 254, such as elastomeric layers or bumps, to grip the anchor 236 when assembling the tab adaptor to the catheter hub 102. Alternatively, a gripping sleeve (not shown) may be provided or secured around the anchor 236 and the gripping sleeve and anchor may be inserted into the receiving socket as a unit to assemble the tab adaptor 160 to the catheter hub 102. The gripping sleeve can be made from any number of rubbery tacky material to increase friction between the sleeve and the wall surfaces of the receiving socket. Still alternatively, the tab adaptor 160 can incorporate a receiving socket and the catheter hub can incorporate a complementary projection for projecting into the socket of the tab adaptor. This arrangement eliminates any pocket that may collect fluids and dust, etc. The tab adaptor can incorporate two receiving sockets or one receiving socket and one anchor.

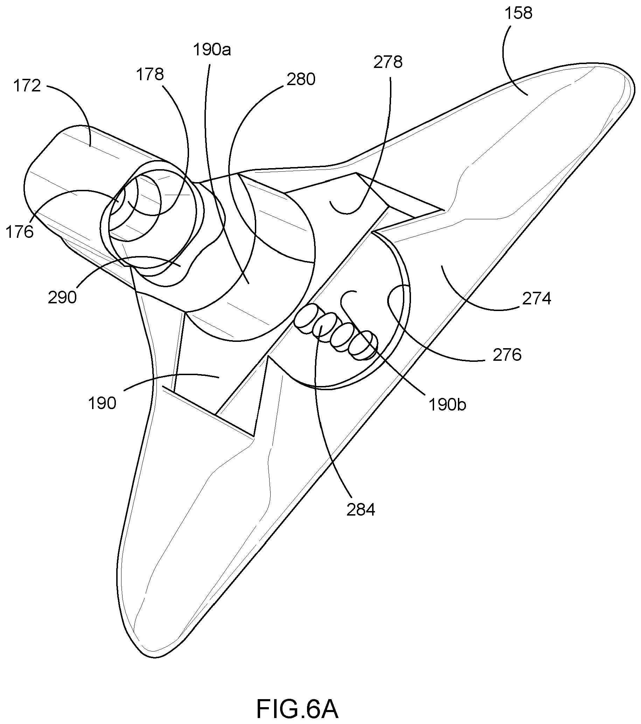

FIGs. 5A, 5B, and 5C are perspective views of the catheter unit 166 of FIGs. 3A-3C with the catheter hub 102, the hub extension 158, and the tab adaptor 160 separated from one another for better viewing their structural features. Refer initially to FIG. 5A, the catheter hub 102 is shown with a hub body 156 having a generally cylindrical main body section, which is conventional. A unitarily formed push tab 133 is provided with the body section, as previously discussed. At the distal end of the hub body 156, a nose section 188 is located having a relatively smaller physical profile than the generally cylindrical main body section. In an example, the nose section 188 has a distal section 258, a mid-section 260, and a base section 262. In an example, the distal section 258 can be generally cylindrical, the mid-section 258 can be frusto-conical in shape, and the base section 262 can be cylindrical or frusto-conical in shape. Preferably, the base section 262 has distinct sides, such as being polygonal in shape. As shown, the base section 262 has four sides with each side having arcuate or curved sub-sections. In other examples, the sides can be straight and can connect to one another via curved sections. Alternatively, the base section can be circular or cylindrical in shape. The nose section 188 is configured to project into the proximal opening 176 of the tip 172 of the hub extension 158. In an example, the bore of the tip 172 of the hub extension and the nose section 188 of the catheter hub have complementary surfaces. The complementary surfaces facilitate alignment and secures the distal end of the catheter hub 102 to the hub extension, such as by physical constraint. The catheter hub is constrained from moving relative to the hub extension by the body of the hub extension from the ready to use position and through the used position, such as being constrained from moving axially within the pocket of the hub extension. The nose section can press against the interior surface of the tip 172 to limit looseness or slack. As shown in FIG. 6A, the shaped base section 262 of the catheter hub 102 can rest against complementary surfaces formed in the distal pocket section 190a of the hub extension 158, such as the contoured section 290 of the distal pocket.

A socket saddle 266 is provided with the hub body 156. In an example, the socket saddle 266 is co-molded, insert-molded, or unitarily molded with the hub body. In other examples, the socket saddle 266 can be separately formed and subsequently attached to the catheter hub, such as be welding, adhesive, snap fit, detents, or combinations thereof. The socket saddle 266 can include two receiving sockets 240, one on each side of the longitudinal axis of the catheter hub 102. Each receiving socket 240 of the socket saddle 266 has a plurality of walls, including end walls 240e and a bottom wall 240d. As shown, each end wall 240e has a curved side edge 270 that matches the exterior curvature of the hub body 156. The bottom wall 240d can be generally flat or planar and extends from the outer sidewall 240c to below or underneath the hub body 156, wherein the bottom wall 240d joins with the bottom wall 240d of the second receiving socket 240. The unitarily formed push tab 133 generally aligns with the proximal end wall 240e of the receiving socket 140 but can vary. In other examples, the push tab 133 can align with the distal end wall 240e or somewhere between the two end walls 240e, 240e. In alternative embodiments, the socket saddle 266 has a structure with open sides or with projections so that no actual sockets are utilized to receive male projections. For example, where a tab adaptor incorporates sockets, the socket saddle can incorporate corresponding or complementary projections to project into the sockets of the tab adaptor. As shown, the socket saddle 266 has a width, measured orthogonally to the length of the catheter hub, that is wider than the diameter or width of the catheter hub. In an embodiment, the socket saddle 266 has sidewalls 240c, 240c that are located radially, and spaced, away from the exterior of the catheter hub.

With reference now to FIG. 5B, an isometric view of the hub extension 158 is shown. As previously discuss, the hub extension 158 comprises a body 164 comprising a tip 172, two wings 198, and a pocket or cavity 190 for accommodating part of the catheter hub 102 and the socket saddle 266. The pocket 190 has a distal pocket section 190a and a proximal pocket section 190b. The two pocket sections have different shapes or geometries. As shown, the distal pocket section 190a is shaped as a partial cylinder defining a cradle for accommodating the distal cylinder portion 156a of the catheter hub 102. The proximal pocket section 190b is generally rectilinear in shape for accommodating the socket saddle 266, which is subjacent part of the hub body 156. In other examples, the shapes of the distal socket section 190a and the proximal socket section 190b can differ and be complementary to the surfaces of the catheter hub.

The proximal pocket section 190b has a proximal wall 274 with an arcuate cutout 276, a distal wall 278 with an arcuate cutout 280, and two sidewalls 282, 282 that form part of the inner edges of the two wings 198. The proximal wall 274 of the proximal pocket section 190b can align with the two trailing edges 202, 202 of the two wings 198, 198. In an example, the two arcuate cutouts 276, 280 are aligned to accommodate a generally round cylinder. The two arcuate cutouts 276, 280 can have different cutout sizes to accommodate for the tapering cylinder of the catheter hub. Because the hub body 156 of the catheter hub 102 tapers in the distal direction, the distal cutout 280 can have a smaller radius than the proximal cutout 276.

A plurality of mating members 284 are provided on the bottom wall 286 of the pocket or cavity 190 for engaging complementary mating members at the bottom of the catheter hub 102, such as the bottom of the socket saddle 266, as further discussed below. The bottom wall 286 can form part of the bottom surface 234 (FIG. 3C) of the hub extension 158. In the present embodiment, the plurality of mating members 284 embody spaced apart projections. The projections can be short spaced apart round stubs. The short spaced apart round stubs 284 can project into corresponding round mating members or holes on the catheter hub 102, or to a structure secured to the catheter hub, to secure the catheter hub to the hub extension 158. As shown, there are four spaced apart stubs that align in a straight line. In other examples, there can be fewer than four spaced apart stubs or greater than four. In still other examples, the shape of the stubs 284 can differ, such as having oval shapes, triangular shapes, square shapes, or rectangular shapes, to name a few non-limiting examples. In yet other examples, the mating members 284 can embody holes or openings for engaging stubs formed at the bottom of the catheter hub, or at the socket saddle attached to the catheter hub. While the mating members 284 are shown arranged in a line along the lengthwise axis of the hub extension 158, the mating members 284 can be arranged differently, such as in a line orthogonal to the lengthwise axis, near the four corners of the proximal pocket section 190b, or randomly situated. The arrangement of the mating members on the catheter hub 102, such as on the socket saddle 266, can be positioned accordingly to engage the mating members 284 on the hub extension.

With reference now to FIG. 5C, a front-bottom perspective view of the tab adaptor 160 is shown. The distal wall 228 and the curved bottom surface 248 of the tab adaptor 160 can clearly be seen. Also shown are the two spaced apart anchors 236, 236, as previously discussed. Also shown is a recessed section 288 formed at the curved bottom surface 248. The recessed section 288 can be incorporated for accommodating the projection of the unitarily formed tab 133 located at the top of the catheter hub 102. If the unitarily formed tab 133 is omitted, the recessed section 288 can also be omitted. The shape of the recessed section 288 can be complementary to the unitarily formed tab 133. The shape of the recessed section 288 can be different and/or larger than required to accommodate the unitarily formed tab 133.

With reference now to FIG. 6A, a rear perspective view of the hub extension 158 of FIG. 5B is shown. The present view more clearly shows the pocket or cavity 190, and more particularly the distal pocket section 190a and the proximal pocket section 190b. A contoured surface 290 is provided in the distal pocket section 190a, adjacent the proximal opening 176 of the tip 172. The contoured surface 290 is complementary to surfaces of the base section 262 of the catheter hub nose section 188. As shown in FIG. 6A, and also in FIG. 5B, the perimeter profile of the two wings is chamfered or rounded to eliminate sharp edges. The wings 198 also have a thickness profile that varies in the radial direction, from the tip of the wing to the base of the wing. In other words, the base of each wing 198 can be thicker than the radial outmost part of the wing. Typical prior art wings have a single thickness along the radial direction, which can cause tenting and sharp size differences between the catheter hub and the wings.

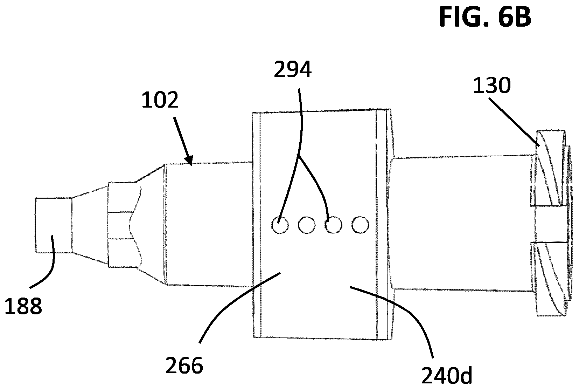

FIG. 6B is a bottom plan view of the catheter hub 102 of FIG. 5A. The surface of the socket saddle 266 is clearly shown having a rectangular profile or perimeter, which is sized and shaped to fit in the proximal pocket section 190a of the hub extension 158, as previously discussed. Also shown are mating members 294 on the socket saddle 266 for mating with the mating members 284 of the proximal pocket section 190b. The present mating members 294 can embody holes or openings for receiving the stubs of the proximal pocket section 190b.

In an embodiment of the present invention, the catheter hub 102 is assembled with a catheter tube and then assembled to the hub extension 158. The catheter tube can first project through the bore or lumen 178 of the tip 172. The nose section 188 is then inserted into the lumen 178 while the hub body 156 is lowered into the pocket 190 so that the round hub body 156 settles into the curved cutout 276 on the proximal wall 274 and the curved cutout 280 on the distal wall 278. As the hub body is lowered, the complementary surfaces of the hub body 156 and the hub extension 158 align, and the two sets of mating members 284, 294 engage to secure the two components together.

Aspects of the present invention therefore comprises a catheter unit comprising a catheter hub having a hub body, a catheter tube extending distally of the hub body, and a proximal female Luer opening. The catheter unit further comprising a hub extension comprising a body with a tip having a distal opening, a proximal opening, and a lumen extending between the two openings, a pocket or cavity located proximally of the tip, and a pair of wings extending laterally of the pocket or cavity, and wherein at least part of the hub body of the catheter hub is located in the pocket or cavity. The catheter unit further comprising a tab adaptor mounted over an upper surface of the hub body. The tab adaptor can have a lower surface that is curved or arcuate for sitting atop the hub body. The lower surface of the tab adaptor can have a recessed section for accommodating a unitarily formed push tab that is formed with the hub body.

The catheter hub can seat inside the pocket or cavity and a proximal section of the catheter hub, including the proximal end opening, and the exterior threads if incorporated, extend proximally of a proximal wall of the hub extension. The proximal wall of the hub extension can have a cutout. The bottom surface of the hub extension can be generally flat or planar. The material of the hub extension can be softer than the material of the catheter hub. A needle can project through the catheter hub and the catheter tube. The needle can attach to a needle hub and the needle hub can be located proximal of the catheter hub.

The tab adaptor can attach to the catheter hub. A socket saddle is attached to the hub body and the tab adaptor is attached to the socket saddle. The attachment can be detents, snap fit, interference fit, friction fit, or combinations thereof. The hub body of the catheter hub can have a nose section and at least part of the nose section can extend through the proximal opening of the tip of the hub extension. The socket saddle can have two receiving sockets. Each receiving socket can have a generally rectilinear shape. In other examples, each socket can have one or more round or oval sockets. Where more than one round or oval socket is utilized, the two or more round or oval sockets can be spaced from one another or can connect or communicate via a channel.

The wings of the hub extension can each have a leading edge and a trailing edge. The leading edge can slope or angled in the proximal direction. Said differently, the leading edge is not orthogonal to a lengthwise axis of the hub extension but angle to slanted in the proximal direction. Each wing can have a perimeter and wherein the perimeter is rounded and smooth, and not formed as a single thickness mold, which is typical in the prior art. In other examples, the hub extension can omit the tapered wings, as further discussed below.

A needle guard can be located inside the catheter hub. The needle guard can have two arms with each arm comprising an elbow and the two elbows expanded by the needle to contact the interior of the catheter hub. Optionally a valve and a valve opener can be located in the catheter hub. The valve and valve opener, if incorporated, can embody any of the structures discussed elsewhere herein.

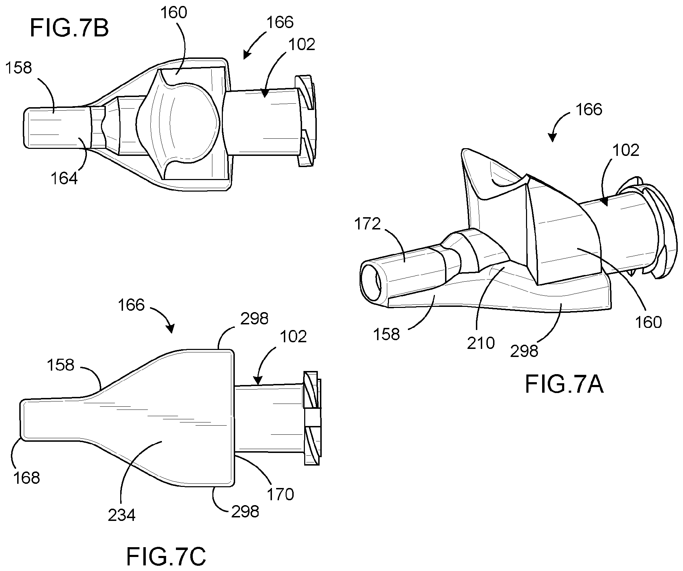

FIGs. 7A, 7B, and 7C show a perspective view, a top view, and a bottom view, respectively, of a catheter unit 166 provided in accordance with further aspects of the invention. The present catheter unit 166 is similar to the catheter units of FIGs. 2-6B, and specifically to FIGs. 3A-3C, with a few exceptions. Like other catheter units discussed elsewhere herein, the catheter unit 166 of the present embodiment comprises a catheter hub 102, a hub extension 158, and a removable tab adaptor 160. However, the present hub extension 158 is not provided with opposing wings 198. Instead, the body 164 of the hub extension 158 terminates adjacent the base section 210 of the body 164. The present hub extension 158 has two side edges 298 defining a maximum width of the hub extension 158, and wherein the width of the present hub extension is less than or smaller than the width of the hub extension 158 with the two opposing wings 198 (FIGs. 3A-3B). However, as shown FIG. 7C, the bottom surface 234 still provides a generally flat planar surface that is substantially larger than the surface of a typical cylindrical catheter hub, and therefore provides a stable base for supporting the catheter hub 102 from rolling compared to prior art hubs with a rounded hub body. The generally flat surface can be seen between the distal end 168 and the proximal end 170 and between the two side edges 298 (FIG. 7C). From a bottom view (FIG. 7C), the hub extension 158 resembles a modified D-shape with a stub projection extending from the curved part of the D-shape. The modified D-shape reduces the height to width ratio of the "D". The hub extension 158 provides a generally flat surface that spans from the nose section of the catheter hub along the length of the catheter hub to about a mid-length of the catheter hub so that the curved or rounded body of the catheter hub does not directly contact the patient.

In an example, the catheter hub 102 and the tab adaptor 160 can be similar to the catheter hub and the tab adaptor of FIGs. 3A-3B, 5A, and 5C. Thus, a needle with a needle hub may be used with the catheter hub as previously discussed. Optionally, a needle guard, a valve, and a valve opener can be used with the catheter hub, as discussed elsewhere herein.

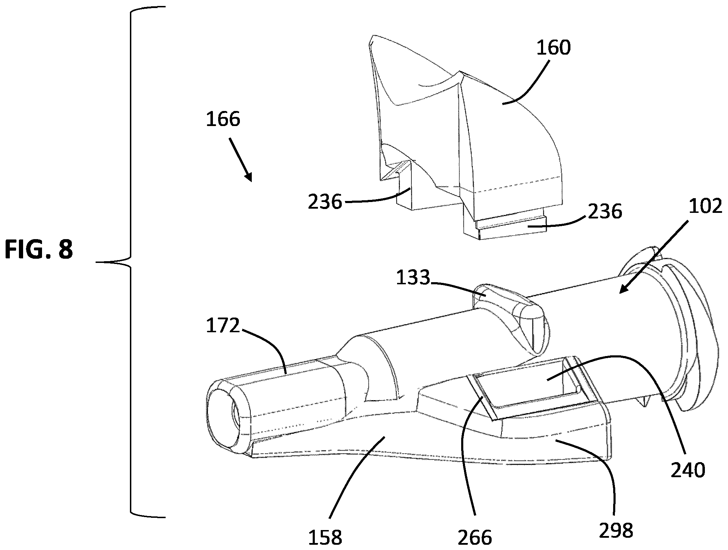

FIG. 8 is a partially exploded perspective view of the catheter unit 166 of FIGs. 7A-7C with the tab adaptor 160 separated from the catheter hub 102, similar to FIG. 4A. The separated position shown is analogous to when vascular access or successful venipuncture has been achieved and the tab adaptor 160 is separated from the catheter hub 102 to then secure the catheter hub to the patient, such as by adhesive or medical dressing. The tab adaptor 160 may engage the socket saddle 266 in the same manner as previously discussed.

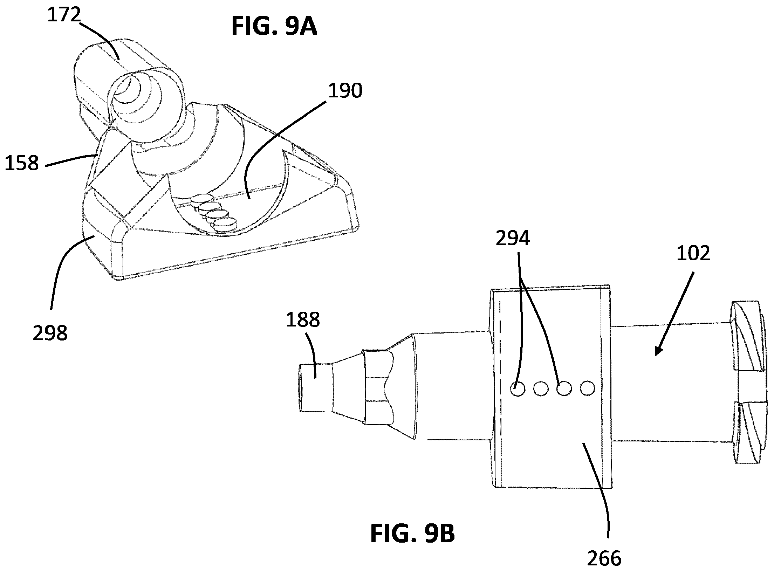

FIGs. 9A is a rear perspective view of the hub extension 158 of FIGs. 7A-7C and FIG. 9B is a bottom plan view of the catheter hub 102 of FIGs. 7A-7C, similar to FIGs. 6A and 6B, respectively. Thus, whereas the hub extension of the embodiment of FIGs. 3A-3C have radially extending wings, the body of the present hub extension terminates with two side edges 298.

FIGs. 10A and 10B are perspective views of an alternative tab adaptor 160 for removably attaching to a catheter hub 102 and/or hub extension 158 of the present invention, such as that of FIGs. 3A-3C and 7A and 7C. In the present tab adaptor 160 embodiment, the adaptor body 214 comprises an upstanding tab section 300 attached to a horizontally positioned tab section or base 302. The underside surface of the base 302 can comprise a dimple or arcuate recess 248 for form fitting around the curvature of the catheter hub.

The spaced apart anchors 236 may comprise spaced apart posts 304. There can be more than one post 304 adjacent each of the two side edges 306 of the base 302. As shown, there are two spaced apart posts adjacent each side edge 306. The posts can project into receiving bores formed with the anchor saddle of the catheter hub or receiving bores formed with the hub extension. The posts 304 can engage corresponding bores to removably engage the tab adaptor 160 to the catheter hub 102 or the hub extension 158. The posts 304 and the complementary receiving bores can be sized and shaped to provide the requisite removable engagement for use to perform vascular access with the disclosed catheter assemblies.

FIGs. 11A and 11B are perspective views of an alternative tab adaptor 160 for removably attaching to a catheter hub 102 and/or hub extension 158 of the present invention, such as that of FIGs. 3A-3C and 7A and 7C. In the present tab adaptor 160 embodiment, the adaptor body 214 resembles a lounge chair. As shown, the adaptor body 214 comprises an upstanding wall section 306, two sidewalls 216, and a base section 308 comprising raised surface features for added gripping. The base section 308 can have a contoured bottom 248 for form fitting around the curvature of the catheter hub. A recessed section 288 is also provided for accommodating the unitarily formed push tab on the catheter hub. The upstanding wall section 306 can have a distal wall surface 228 comprising raised surface features for added gripping.

Two spaced apart anchors 236 are provided at the bottom of the tab adaptor. Each of the two anchors 236 resembles two posts connected to one another by a web. The anchors 236 can project into receiving bores formed with the anchor saddle of the catheter hub or receiving bores formed with the hub extension. The anchors 236 can engage corresponding receptacles or sockets to removably engage the tab adaptor 160 to the catheter hub 102 or the hub extension 158. The anchors 236 and the complementary receiving sockets can be sized and shaped to provide the requisite removable engagement for use to perform vascular access with the disclosed catheter assemblies.

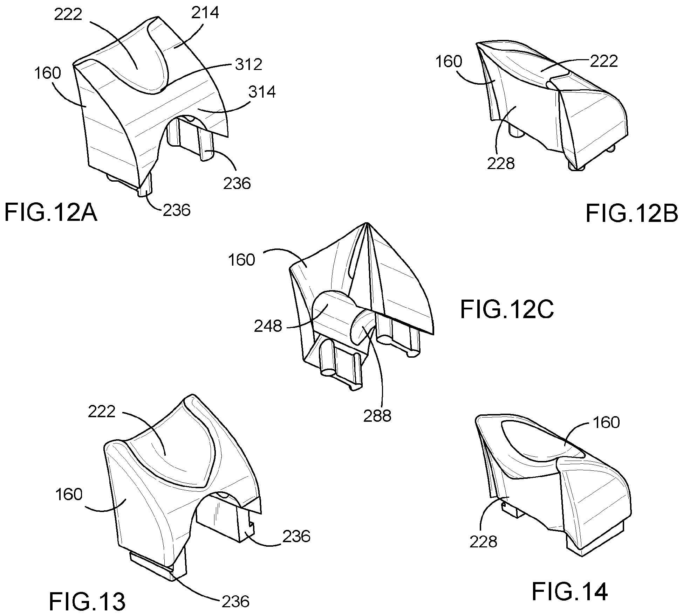

FIGs. 12A, 12B, and 12C are perspective views of an alternative tab adaptor 160 for removably attaching to a catheter hub 102 and/or hub extension 158 of the present invention, such as that of FIGs. 3A-3C and 7A and 7C. The present tab adaptor 160 resembles the tab adaptor of FIGs. 4A, 5C, and 8 with a few exceptions. As shown, the adaptor body 214 comprises a concave section or dimple 222 located on a downward slope, which slopes in the proximal direction. The proximal end most point 312 of the dimple 222 terminates before the end of the sloped surface, and before the proximally facing wall surface 314. The bottom of the tab adaptor can include a contoured bottom 248 for form fitting around the curvature of the catheter hub and a recessed section 288 for accommodating the unitarily formed push tab on the catheter hub.

Two spaced apart anchors 236 are provided at the bottom of the tab adaptor. Each of the two anchors 236 resemble two posts connected to one another by a web, similar to that of FIGs. 11A and 11B. The anchors 236 can project into receiving bores formed with the anchor saddle of the catheter hub or receiving bores formed with the hub extension. The anchors 236 can engage corresponding receptacles or sockets to removably engage the tab adaptor 160 to the catheter hub 102 or the hub extension 158. The anchors 236 and the complementary receiving sockets can be sized and shaped to provide the requisite removable engagement for use to perform vascular access with the disclosed catheter assemblies.

FIGs. 13 and 14 are perspective views of an alternative tab adaptor 160 for removably attaching to a catheter hub 102 and/or hub extension 158 of the present invention, such as that of FIGs. 3A-3C and 7A and 7C. The present tab adaptor 160 resembles the tab adaptor of FIGs. 3B, 4A, 5C, and 8 with a few exceptions. As shown, the adaptor body 214 comprises a concave or dimple 222 located on a downward slope, which slopes in the proximal direction. The proximal end most point 312 of the dimple 222 terminates near the end or bottom of the sloped surface. The end most point 312 is somewhat more angled or pointed than the curved end point of the same dimple of FIG. 3B.

The bottom of the tab adaptor can include a contoured bottom 248 for fitting around the curvature of the catheter hub and a recessed section 288 for accommodating the unitarily formed push tab on the catheter hub. The two anchors 236, 236 can be similar to that of FIGs. 3B, 4A, 5C, and 8.

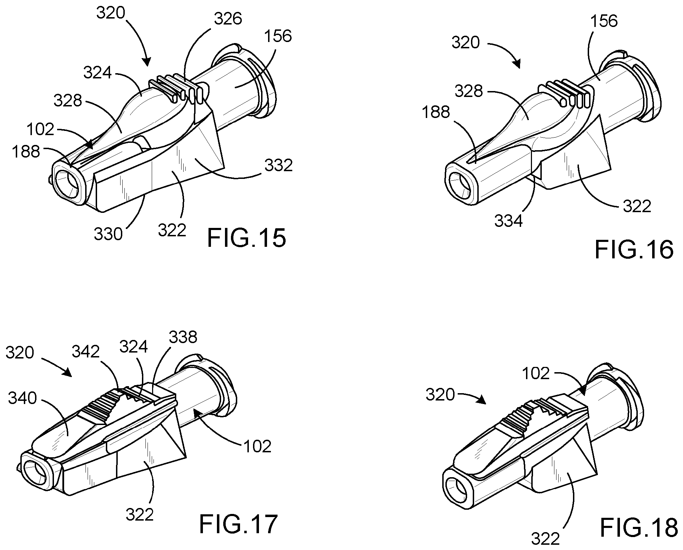

FIG. 15 is a perspective view of an alternative catheter unit 320 with a base unit 322 and FIG. 16 is a similar catheter unit 320 but with a relatively shorter base unit 322. With reference initially to FIG. 15, the catheter unit 320 is shown with a catheter hub 102 and a unitarily formed push tab 324. In the present embodiment, the unitarily formed push tab 324 has a roughened portion 326 and a distal portion 328. The roughened portion 326 can comprise a plurality of spaced ribs or fins for improved finger grip. Alternatively, the roughened portion 326 can embody other roughened surfaces, such as raised bumps or dimples. The distal portion 324 has a body that is generally rounded and tapered to form a smooth transition between the tab and the hub body 326 of the catheter hub 102. The nose end or nose portion 188 of the catheter hub 102 has a catheter tube projecting therefrom (not shown), which can attach to the interior of the catheter hub with a ferrule or metal bushing. A valve, a valve opener, and a needle guard or tip protector may be located inside the catheter hub, as previously discussed.

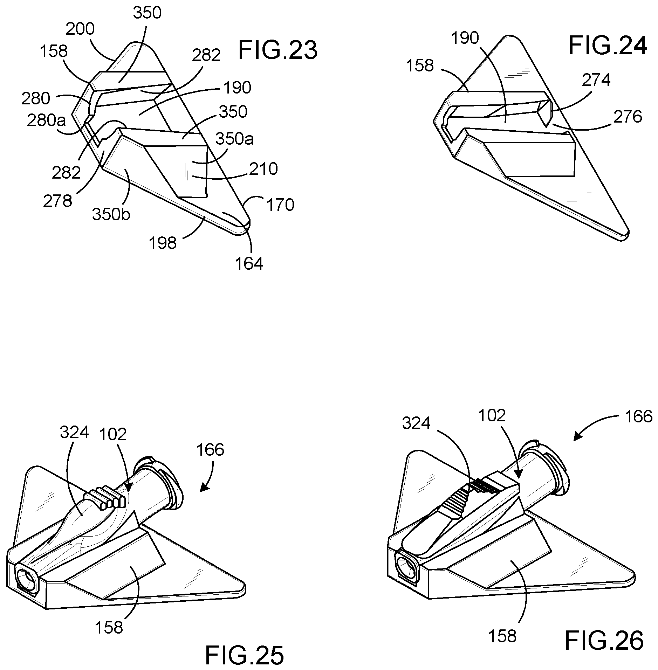

A base unit 322 having a body with a cavity for receiving the catheter hub 102 is shown. The catheter hub 102 can seat within the cavity of the base unit 322 and can engage the base unit using detents or snap fittings. The base unit 322 can have a length that extends from about the nose section to about the proximal end of the push tab 324. The base unit 322 can have a flat base bottom 330. The flat base bottom 330 provides a flat surface with the patient's skin to minimize rotation or movement of the catheter hub during connector change out. The base unit 322 can have sidewalls (only one shown) that can be flat and vertical, but can alternatively be arcuate, flared, or tapered. In still other examples, the catheter hub 102 and the base unit 322 may be unitarily formed. When separately formed, the catheter hub and the base unit of FIG. 15 may be made with similar materials as the catheter hubs and hub extensions discussed elsewhere herein. In some embodiments, the catheter unit 320, including the base unit 322, can seat within a cavity or pocket of a hub extension so that the flat bottom surface of the hub extension seats against the patient, as shown and further discussed below in FIGs. 25 and 26.

The catheter unit 320 of FIG. 16 is similar to the catheter unit 320 of FIG. 15 but wherein the base unit 322 is relatively shorter. In the present embodiment, the base unit 322 has a length that is approximately half of the base unit 322 of FIG. 15. In the example shown, the distal end 334 of the base unit 322 is recessed from the nose section 188 of the catheter hub. The distal end 334 can be located at about the half-way mark of the hub body 156, below the distal portion 328 of the push tab and can extend to approximately the roughened portion of the push tab.

FIG. 17 is a perspective view of an alternative catheter unit 320 that is similar to the catheter unit of FIG. 15. However, in the present embodiment, the push tab 324 has a structure with a sloped proximal portion 338 and a sloped distal portion 340. The two sloped portions meet along a crest 342. The two sloped portions can both have roughened surfaces, such as having bumps or projections.

The catheter unit 320 of FIG. 18 is similar to the catheter unit of FIG. 17, but with a shortened base unit 322.

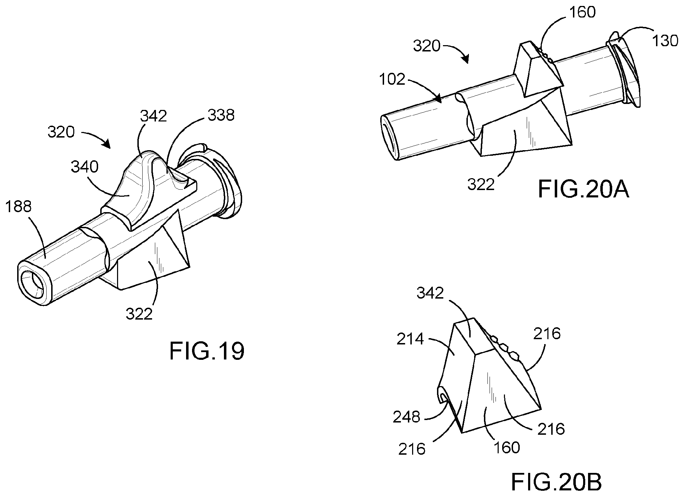

FIG. 19 is a perspective view of an alternative catheter unit 320 that is similar to the catheter unit of FIG. 18. However, in the present embodiment, the sloped distal portion 340 of the push tab 324 is shortened and has a relatively steep slope. The two sloped portions meet along a crest 342, which is more pointed than elongated compared to the crest of FIG. 18. The two sloped portions can both have roughened surfaces, such as having bumps or projections. In other examples, the base unit 322 of the present catheter unit 320 can extend longer from just proximal of the end of the nose section 188 to about the proximal sloped portion 338.

In alternative embodiments, the push tabs of the catheter units of FIGs. 15-19 may be separately formed from the catheter hubs and removably attachable therewith, such as with detents or snap fit.

FIG. 20A is a catheter unit 320 provided in accordance with still yet further aspects of the invention, which comprises catheter hub 102, a tab adaptor 160, and a base unit 322. The present catheter unit 320 has removable sub-components, similar to the catheter unit 166 of FIGs. 3A-3C and 7A-7C. For example, the catheter hub 102 can removably engage with the base unit 322 and the tab adaptor 160 can removably engage with the catheter hub 102. The tab adaptor 160 is shown in FIG. 20B separated from the catheter hub 102. In some examples, the tab adaptor 160 is resilient and flexible and can be non-removable from the catheter hub.

The base unit 322 of FIG. 20A can be similar to the base unit of FIG. 16. However, a longer base unit, such as that of FIG. 15, may be practiced with the catheter hub 102. The catheter hub 102 can resemble a standard catheter hub, one without integrated wings. However, the hub 102 is modified to engage the base unit 322, such as by detents, snap fit, interference fit, adhesive, or by welding.

The tab adaptor 160 has a body 214 that resembles a modified pyramid, with multiple sides 216 and a ridge 342. The various sides 216 can be generally flat or planar and sloped. The slopes of the different sides can be the same or different. In the example shown, the slope of the proximal side is less than the slope of the distal side. A plurality of bumps or projections can be incorporated on the surface of the proximal side for improved gripping. An arcuate lower surface 248 is provided for fitting over the exterior of the catheter hub. The tab adaptor 160 can removably engage with the catheter hub, such as with detents, interference fit or snap fit. Although not shown, a recessed section, similar to the recessed section 288 of FIG. 11Bb, can be incorporated to accommodate the unitarily formed push tab formed with the catheter hub.

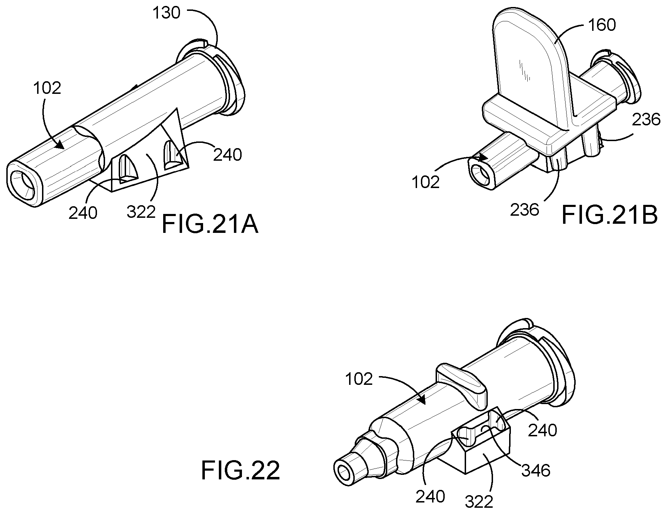

FIG. 21A shows a catheter hub 102 secured to a base unit 322 comprising receiving sockets 240 for receiving and engaging anchors of a tab adaptor, such as the tab adaptor 160 shown in FIG. 21B. The catheter hub 102 is shown without unitarily formed push tab. The tab adaptor 160 can be similar to the tab adaptor 160 of FIGs. 10A and 10B. The tab adaptor 160 can have a flat or rounded bottom surface, but without the need for a recessed cavity since the catheter hub 102 does not incorporate any push tab. The spaced apart posts 236 on the tab adaptor of FIG. 21B can engage the spaced apart receiving sockets 240. The receiving sockets 240 can have contoured surfaces for mating engagement with contoured surfaces of the anchors, for removable engagement between the two. The base unit 322 can otherwise resemble the base unit of FIG. 16.

FIG. 22 shows a catheter hub 102 secured to a base unit 322 comprising receiving sockets 240 for receiving and engaging anchors of a tab adaptor, such as the tab adaptor 160 of FIGs. 11A and 11B. In the present embodiment, the two sockets 240 on each side of the socket saddle are generally round and a narrow channel 346 connects the two sockets 240. The two sockets and the channel 346 are sized and shaped to engage corresponding anchors 236 on a tab adaptor. The catheter hub 102 can be similar to the catheter hub of FIGs. 3A-3C, 4A and 7A-7C. The catheter hub 102 can engage the base unit 322 using detents, press fit, or interference fit. Alternatively, the base unit can be glued, bonded, or welded to the catheter hub. In still other examples, the base unit can be molded with the catheter hub, such as over-molding. The catheter hub 102 and the base unit 322 can optionally engage a hub extension so that the hub extension, with wings, can rest against the patient. Exemplary catheter hub and base unit situated in a pocket or cavity of a hub extension is shown in FIGs. 25 and 26 and further discussed below.

FIG. 23 is a perspective view of a hub extension 158 provided in accordance with further aspects of the invention. The present hub extension 158 is similar to hub extensions discussed elsewhere herein, including the hub extensions of FIGs. 3A-3B, 4A, 5B, 6A, and 9A, among others. However, the present hub extension 158 does not incorporate a similar tip 172 (FIGs. 3A-3C). As shown, the present hub extension 158 comprises a body 164 comprising a pair of wings 198 extending laterally of a pocket or cavity 190. As shown, two mounds 350, 350 are provide atop the wings 198. The two mounds 350, 350 define the pocket or cavity 190. Each mound can comprise a block comprising a plurality of wall surfaces. The wall surfaces can be generally flat and can be angled to one another. As shown, two angled or ramped surfaces 350a, 350b are incorporated. Each of the side angled or ramped surface 350a on the two wings 198 gradually decreases the height profile of the mount 350 so that when adhesive dressing is applied, tenting is reduced. Similarly, each of the front angled or ramped surface 350b on the two wings 198 gradually decreases the height profile of the mount 350 at the front so that when adhesive dressing is applied, tenting is reduced.

In the embodiment shown, a distal wall 278 is provided with a cutout 280 for accommodating the generally round profile of the catheter hub 102. In an example, the cut-out 280 includes an upper rounded portion and a lower straight section 280a. In other examples, the cut-out can have different shapes. The shapes can depend on the shape of the catheter hub and/or base unit, as shown in FIGs. 25 and 26. Said differently, the cut-out can be sized and shape to matingly receive the catheter hub and/or the base unit.

In an example, the proximal end of the cavity or pocket 190 is open, or not obstructed. The open configuration is configured to receive a generally wide and flat base unit 322. As shown, the cavity sidewalls 282 terminate at respective proximal ends without any other wall or structure to provide for the open configuration. In other examples, such as shown in FIG. 25, the proximal end of the pocket or cavity is confined by a proximal wall 274 with a cutout 276. The cutout is sized and shaped to accommodate the catheter hub and/or the base unit.

FIG. 25 shows a catheter unit 166 provided in accordance with further aspects of the invention. As shown, the catheter unit 166 comprises a catheter hub 102, a push tab 324, and a hub extension 158. In a particular example, the catheter hub 102 and the push tab 324 can be similar to the push tab and catheter hub of FIG. 15 or FIG. 16. The hub extension 158 can be similar to the hub extension of FIG. 24 or FIG. 25, depending on the length of the base unit 322 secured to the catheter hub 102.

FIG. 26 shows a catheter unit 166 provided in accordance with further aspects of the invention. As shown, the catheter unit 166 comprises a catheter hub 102, a push tab 324, and a hub extension 158. In a particular example, the catheter hub 102 and the push tab 324 can be similar to the push tab and catheter hub of FIG. 17 or FIG. 18. The hub extension 158 can be similar to the hub extension of FIG. 24 or FIG. 25, depending on the length of the base unit 322 secured to the catheter hub 102.

Any of the catheter units 166, 320 described herein are understood to include a catheter tube, a needle, a needle hub, and optionally one or more of a needle guard, a valve, a valve opener, and a third housing for housing the needle guard, as described elsewhere herein.

Methods of making catheter assemblies and catheter units and components thereof as described elsewhere herein are within the scope of the present invention.

Although limited embodiments of needle assemblies and their components have been specifically described and illustrated herein, many modifications and variations will be apparent to those skilled in the art. Accordingly, it is to be understood that the needle assemblies and their components constructed according to principles of the disclosed device, system, and method may be embodied other than as specifically described herein. The invention defined in the following claims.

## Claims

1. A catheter assembly (100) comprising:
a catheter hub (102) comprising a hub body (156) with a width, the hub body (156) having an upper surface (156a) located, elevation-wise, above a lower surface (156b);
a catheter tube (105) having a length and a lumen extending distally of a nose section (188) of the catheter hub (102);
a needle (106) attached to a needle hub (104) and having a needle tip (108) extending through the hub body (156) and the lumen of the catheter tube (105) in a ready to use position;
a hub extension (158) having a body (164) with a pocket (190) having at least part of the hub body (156) and the saddle socket (266) located in the pocket (190) and constrained from moving relative to the hub extension (158) by the body (164); and
a tab adaptor (160) surrounding at least part of the upper surface (156a) of the catheter hub (102), the tab adaptor (160) having two sidewalls (216, 216) defining a width that is wider than the width of the hub body (156), wherein the tab adaptor (160) is separable from the catheter hub (102),
**characterized in that**
the catheter hub (102) comprises a saddle socket (266) having wall surfaces defining a width that is wider than the width of the hub body (156), wherein the saddle socket (266) is attached to the hub body (156) and the tap adaptor (160) is attached to the saddle socket (266).

2. The catheter assembly (100) of claim 1, wherein the hub extension (158) comprises a pair of wings (198, 198) extending laterally of the pocket (190).

3. The catheter assembly (100) of claim 1 or 2, further comprising a tip (172) having a lumen (178) and wherein at least part of the nose section (188) of the catheter hub (102) is located in the lumen (178).

4. The catheter assembly (100) according to any one of claims 1 to 3, wherein the hub extension (158) has a flat bottom surface (234).

5. The catheter assembly (100) according to any one of claims 1 to 4, wherein the hub extension (158) is made from a material that is softer and more flexible than a material used to form the hub body (156) of the catheter hub (102).

6. The catheter assembly (100) according to any one of claims 1 to 5, wherein the tab adaptor (160) has a lower surface (248) and two spaced apart anchors (236, 236) extending from the lower surface (248).

7. The catheter assembly (100) of claim 6, wherein the socket saddle (266) has two spaced apart sockets (240, 240) and wherein the two anchors (236, 236) engage the two sockets (240, 240).

8. The catheter assembly (100) according to any one of claims 1 to 7, wherein the pocket (190) has a bottom wall (286) comprising a plurality of mating members (284).

9. The catheter assembly (100) of claim 8, wherein the saddle socket (266) comprises a plurality of mating members (294), and wherein the mating members (284) at the bottom wall (286) of the pocket (190) engage the mating members (294) on the saddle socket (266).

10. The catheter assembly (100) according to any one of claims 1 to 9, further comprising a needle guard (122) located in an interior cavity (118) of the hub body (156).

11. The catheter assembly (100) according to any one of claims 1 to 10, further comprising a valve and a valve opener located in an interior cavity (118) of the catheter hub (102).

12. The catheter assembly (100) according to any one of claims 1 to 11, wherein the hub extension (158) has a leading edge (200) and a trailing edge (202) that are not parallel.

13. The catheter assembly (100) according to any one of claims 1 to 12, wherein the hub extension (158) is D-shaped with a projection extending axially of a curved section of the D-shaped.

14. The catheter assembly (100) according to any one of claims 1 to 13, wherein the hub extension (158) has two side edges (298, 298) defining a maximum width of the hub extension (158).

15. The catheter assembly (100) of claim 14, wherein the maximum width is wider than the width of the tab adaptor (160) and the width the hub body (156).

16. The catheter assembly (100) according to any one of claims 1 to 15, wherein the catheter hub (102) has a push tab (133) and the tab adaptor (160) has a recessed section (288) at a lower surface (248) located around the push tab (133).

17. The catheter assembly (100) according to any one of claims 1 to 16, wherein the tab adaptor (160) has a concave surface (222) located between the two sidewalls (216, 216).

18. The catheter assembly (100) according to any one of claims 1 to 17, wherein the hub extension (158) has a tip (172) comprising a distal opening (174) and a proximal opening (176), and wherein the distal opening (174) has a ramped surface (182) and the proximal opening (176) having the nose section (188) of the catheter hub (102) located therein.

19. A method of manufacturing a catheter assembly (100) comprising the steps:
forming a catheter hub (102) comprising a hub body (156) with a width, the hub body (156) having an upper surface (156a) located, elevation-wise, above a lower surface (156b);
attaching a catheter tube (105) having a length and a lumen to a nose section (188) of the catheter hub (102);
extending a needle (106) attached to a needle hub (104) and having a needle tip (108) through the hub body (156) and the lumen of the catheter tube (105) in a ready to use position; and
attaching a hub extension (158) having a body (164) with a pocket (190) to the catheter hub (102) by placing at least part of the hub body (156) and the saddle socket (266) in the pocket (190) and constraining the catheter hub (102) from moving relative to the hub extension (158)
and placing a tab adaptor (160) over at least part of the upper surface (156a) of the catheter hub (102), the tab adaptor (160) having two sidewalls (216, 216) defining a width that is wider than the width of the hub body (156), wherein the tab adaptor (160) is separable from the catheter hub (102),
**characterized in that**
the catheter hub (102) comprises a saddle socket (266) having wall surfaces defining a width that is wider than the width of the hub body (156), wherein the saddle socket (266) is attached to the hub body (156) and the tap adaptor (160) is attached to the saddle socket (266).

## Patentansprüche

1. Katheteranordnung (100), umfassend:
einen Katheteransatz (102), umfassend einen Ansatzkörper (156) mit einer Breite, wobei der Ansatzkörper (156) eine obere Oberfläche (156a) aufweist, die sich in der Höhe über einer unteren Oberfläche (156b) befindet;
eine Katheterröhre (105), die eine Länge und ein Lumen aufweist, der sich distal eines Nasenabschnitts (188) des Katheteransatzes (102) erstreckt;
eine Nadel (106), die an einem Nadelansatz (104) befestigt ist und eine Nadelspitze (108) aufweist, die sich in einer gebrauchsfertigen Position durch den Ansatzkörper (156) und das Lumen der Katheterröhre (105) erstreckt;
eine Ansatzverlängerung (158), die einen Körper (164) mit einer Tasche (190) aufweist, die mindestens einen Teil des Ansatzkörpers (156) und der Sattelmuffe (266) aufweist, die sich in der Tasche (190) befindet und durch den Körper (164) an einer Bewegung relativ zu der Ansatzverlängerung (158) gehindert wird; und
einen Laschenadapter (160), der mindestens einen Teil der oberen Oberfläche (156a) des Katheteransatzes (102) umgibt, wobei der Laschenadapter (160) zwei Seitenwände (216, 216) aufweist, die eine Breite definieren, die breiter als die Breite des Ansatzkörpers (156) ist, wobei der Laschenadapter (160) von dem Katheteransatz (102) trennbar ist,
**dadurch gekennzeichnet, dass**
der Katheteransatz (102) eine Sattelmuffe (266) umfasst, die Wandoberflächen aufweist, die eine Breite definieren, die breiter als die Breite des Ansatzkörpers (156) ist, wobei die Sattelmuffe (266) an dem Ansatzkörper (156) befestigt ist und der Laschenadapter (160) an der Sattelmuffe (266) befestigt ist.

2. Katheteranordnung (100) nach Anspruch 1, wobei die Ansatzverlängerung (158) ein Paar Flügel (198, 198) umfasst, die sich lateral der Tasche (190) erstrecken.

3. Katheteranordnung (100) nach Anspruch 1 oder 2, ferner umfassend eine Spitze (172), die ein Lumen (178) aufweist, und wobei sich mindestens ein Teil des Nasenabschnitts (188) des Katheteransatzes (102) in dem Lumen (178) befindet.

4. Katheteranordnung (100) nach einem der Ansprüche 1 bis 3, wobei die Ansatzverlängerung (158) eine flache Bodenoberfläche (234) aufweist.

5. Katheteranordnung (100) nach einem der Ansprüche 1 bis 4, wobei die Ansatzverlängerung (158) aus einem Material besteht, das weicher und flexibler als ein Material ist, das verwendet wird, um den Ansatzkörper (156) der Katheteransatz (102) auszubilden.

6. Katheteranordnung (100) nach einem der Ansprüche 1 bis 5, wobei der Laschenadapter (160) eine untere Oberfläche (248) und zwei voneinander beabstandete Anker (236, 236) aufweist, die sich von der unteren Oberfläche (248) erstrecken.

7. Katheteranordnung (100) nach Anspruch 6, wobei der Muffensattel (266) zwei voneinander beabstandete Muffen (240, 240) aufweist und wobei die zwei Anker (236, 236) die zwei Muffen (240, 240) in Eingriff nehmen.

8. Katheteranordnung (100) nach einem der Ansprüche 1 bis 7, wobei die Tasche (190) eine Bodenwand (286) aufweist, umfassend eine Vielzahl von Passelementen (284).

9. Katheteranordnung (100) nach Anspruch 8, wobei die Sattelmuffe (266) eine Vielzahl von Passelementen (294) umfasst und wobei die Passelemente (284) an der Bodenwand (286) der Tasche (190) die Passelemente (294) an der Sattelmuffe (266) in Eingriff nehmen.

10. Katheteranordnung (100) nach einem der Ansprüche 1 bis 9, ferner umfassend einen Nadelschutz (122), der sich in einem inneren Hohlraum (118) des Ansatzkörpers (156) befindet.

11. Katheteranordnung (100) nach einem der Ansprüche 1 bis 10, ferner umfassend ein Ventil und einen Ventilöffner, die sich in einem inneren Hohlraum (118) der Katheteransatz (102) befinden.

12. Katheteranordnung (100) nach einem der Ansprüche 1 bis 11, wobei die Ansatzverlängerung (158) eine Vorderkante (200) und eine Hinterkante (202) aufweist, die nicht parallel sind.

13. Katheteranordnung (100) nach einem der Ansprüche 1 bis 12, wobei die Ansatzverlängerung (158) D-förmig mit einem Vorsprung ist, der sich axial von einem gekrümmten Abschnitt der D-Form erstreckt.

14. Katheteranordnung (100) nach einem der Ansprüche 1 bis 13, wobei die Ansatzverlängerung (158) zwei Seitenkanten (298, 298) aufweist, die eine maximale Breite der Ansatzverlängerung (158) definieren.

15. Katheteranordnung (100) nach Anspruch 14, wobei die maximale Breite breiter als die Breite des Laschenadapters (160) und die Breite des Ansatzkörpers (156) ist.

16. Katheteranordnung (100) nach einem der Ansprüche 1 bis 15, wobei der Katheteransatz (102) eine Drucklasche (133) aufweist und der Laschenadapter (160) einen vertieften Abschnitt (288) an einer unteren Oberfläche (248) aufweist, die sich um die Drucklasche (133) herum befindet.

17. Katheteranordnung (100) nach einem der Ansprüche 1 bis 16, wobei der Laschenadapter (160) eine konkave Oberfläche (222) aufweist, die sich zwischen den zwei Seitenwänden (216, 216) befindet.

18. Katheteranordnung (100) nach einem der Ansprüche 1 bis 17, wobei die Ansatzverlängerung (158) eine Spitze (172) aufweist, umfassend eine distale Öffnung (174) und eine proximale Öffnung (176), und wobei die distale Öffnung (174) eine Rampenoberfläche (182) aufweist und die proximale Öffnung (176) den Nasenabschnitt (188) der Katheteransatz (102) aufweist, der sich darin befindet.

19. Verfahren zum Herstellen einer Katheteranordnung (100), umfassend die Schritte:
Ausbilden einer Katheteransatz (102), umfassend einen Ansatzkörper (156) mit einer Breite, wobei der Ansatzkörper (156) eine obere Oberfläche (156a) aufweist, die sich in der Höhe über einer unteren Oberfläche (156b) befindet;
Befestigen einer Katheterröhre (105), die eine Länge und ein Lumen aufweist, an einem Nasenabschnitt (188) des Katheteransatzes (102);
Erstrecken einer Nadel (106), die an einem Nadelansatz (104) befestigt ist und eine Nadelspitze (108) aufweist, durch den Ansatzkörper (156) und das Lumen der Katheterröhre (105) in einer gebrauchsfertigen Position; und
Befestigen einer Ansatzverlängerung (158), die einen Körper (164) mit einer Tasche (190) aufweist, an dem Katheteransatz (102), durch ein Platzieren mindestens eines Teils des Ansatzkörpers (156) und der Sattelmuffe (266) in der Tasche (190) und Hindern des Katheteransatzes (102) daran, sich relativ zu der Ansatzverlängerung (158) zu bewegen
und Platzieren eines Laschenadapters (160) über mindestens einem Teil der oberen Oberfläche (156a) des Katheteransatzes (102), wobei der Laschenadapter (160) zwei Seitenwände (216, 216) aufweist, die eine Breite definieren, die breiter als die Breite des Ansatzkörpers (156) ist, wobei der Laschenadapter (160) von dem Katheteransatz (102) trennbar ist,
**dadurch gekennzeichnet, dass**
der Katheteransatz (102) eine Sattelmuffe (266) umfasst, die Wandoberflächen aufweist, die eine Breite definieren, die breiter als die Breite des Ansatzkörpers (156) ist, wobei die Sattelmuffe (266) an dem Ansatzkörper (156) befestigt ist und der Laschenadapter (160) an der Sattelmuffe (266) befestigt ist.

## Revendications

1. Ensemble cathéter (100), comprenant :
une embase de cathéter (102) comprenant un corps d'embase (156) avec une largeur, le corps d'embase (156) ayant une surface supérieure (156a) située, en élévation, au-dessus d'une surface inférieure (156b) ;
un tube de cathéter (105) ayant une longueur et une lumière s'étendant distalement d'une section de nez (188) de l'embase de cathéter (102) ;
une aiguille (106) fixée à une embase d'aiguille (104) et ayant une pointe d'aiguille (108) s'étendant à travers le corps d'embase (156) et la lumière du tube du cathéter (105) dans une position prête à l'emploi ;
une extension d'embase (158) ayant un corps (164) avec une poche (190) ayant au moins une partie du corps d'embase (156) et du support en selle (266) située dans la poche (190) et contrainte dans son mouvement par rapport à l'extension d'embase (158) par le corps (164) ; et
un adaptateur à languettes (160) entourant au moins une partie de la surface supérieure (156a) de l'embase de cathéter (102), l'adaptateur à languettes (160) ayant deux parois latérales (216, 216) définissant une largeur supérieure à la largeur du corps d'embase (156), dans lequel l'adaptateur à languettes (160) peut être séparé de l'embase de cathéter (102),
**caractérisé en ce que**
l'embase de cathéter (102) comprend un support en selle (266) ayant des surfaces de paroi définissant une largeur supérieure à la largeur du corps d'embase (156), dans lequel le support en selle (266) est fixé au corps d'embase (156) et l'adaptateur à languettes (160) est fixé au support en selle (266).

2. Ensemble cathéter (100) selon la revendication 1, dans lequel l'extension d'embase (158) comprend une paire d'ailettes (198, 198) s'étendant latéralement de la poche (190).

3. Ensemble cathéter (100) selon la revendication 1 ou 2, comprenant en outre une pointe (172) ayant une lumière (178) et dans lequel au moins une partie de la section de nez (188) de l'embase de cathéter (102) est située dans la lumière (178).

4. Ensemble cathéter (100) selon l'une quelconque des revendications 1 à 3, dans lequel l'extension d'embase (158) a une surface de fond plate (234).

5. Ensemble cathéter (100) selon l'une quelconque des revendications 1 à 4, dans lequel l'extension de l'embout (158) est fabriquée à partir d'un matériau plus souple et plus flexible que le matériau utilisé pour former le corps d'embase (156) de l'embase de cathéter (102).

6. Ensemble cathéter (100) selon l'une quelconque des revendications 1 à 5, dans lequel l'adaptateur à languettes (160) a une surface inférieure (248) et deux ancrages espacées (236, 236) s'étendant à partir de la surface inférieure (248).

7. Ensemble cathéter (100) selon la revendication 6, dans lequel le support en selle (266) a deux supports espacés (240, 240) et dans lequel les deux ancrages (236, 236) viennent en prise dans les deux supports (240, 240).

8. Ensemble cathéter (100) selon l'une quelconque des revendications 1 à 7, dans lequel la poche (190) a une paroi de fond (286) comprenant une pluralité d'éléments d'accouplement (284).

9. Ensemble cathéter (100) selon la revendication 8, dans lequel le support en selle (266) comprend une pluralité d'éléments d'accouplement (294), et dans lequel les éléments d'accouplement (284) au niveau de la paroi de fond (286) de la poche (190) viennent en prise avec les éléments d'accouplement (294) du support en selle (266).

10. Ensemble cathéter (100) selon l'une quelconque des revendications 1 à 9, comprenant en outre une protection d'aiguille (122) située dans une cavité intérieure (118) du corps d'embase (156).

11. Ensemble cathéter (100) selon l'une quelconque des revendications 1 à 10, comprenant en outre une valve et un ouvreur de valve situés dans une cavité intérieure (118) de l'embase de cathéter (102).

12. Ensemble cathéter (100) selon l'une quelconque des revendications 1 à 11, dans lequel l'extension d'embase (158) a un bord d'attaque (200) et un bord de fuite (202) qui ne sont pas parallèles.

13. Ensemble cathéter (100) selon l'une quelconque des revendications 1 à 12, dans lequel l'extension d'embase (158) est en forme de D avec une saillie s'étendant axialement depuis une section incurvée de la forme en D.

14. Ensemble cathéter (100) selon l'une quelconque des revendications 1 à 13, dans lequel l'extension d'embase (158) a deux bords latéraux (298, 298) définissant une largeur maximale de l'extension d'embase (158).

15. Ensemble cathéter (100) selon la revendication 14, dans lequel la largeur maximale est plus large que la largeur de l'adaptateur à languettes (160) et la largeur du corps d'embase (156).

16. Ensemble cathéter (100) selon l'une quelconque des revendications 1 à 15, dans lequel l'embase de cathéter (102) a une languette de poussée (133) et l'adaptateur à languettes (160) a une section en creux (288) au niveau d'une surface inférieure (248) située autour de la languette de poussée (133).

17. Ensemble cathéter (100) selon l'une quelconque des revendications 1 à 16, dans lequel l'adaptateur à languettes (160) a une surface concave (222) située entre les deux parois latérales (216, 216).

18. Ensemble cathéter (100) selon l'une quelconque des revendications 1 à 17, dans lequel l'extension d'embase (158) a une pointe (172) comprenant une ouverture distale (174) et une ouverture proximale (176), et dans lequel l'ouverture distale (174) a une surface inclinée (182) et l'ouverture proximale (176) ayant la section de nez (188) de l'embase de cathéter (102) située à l'intérieur de celle-ci.

19. Procédé de fabrication d'un ensemble cathéter (100) comprenant les étapes consistant à :
former une embase de cathéter (102) comprenant un corps d'embase (156) avec une largeur, le corps d'embase (156) ayant une surface supérieure (156a) située, en élévation, au-dessus d'une surface inférieure (156b) ;
fixer un tube de cathéter (105) ayant une longueur et une lumière à une section de nez (188) de l'embase de cathéter (102) ;
étendre une aiguille (106) fixée à une embase d'aiguille (104) et ayant une pointe d'aiguille (108) à travers le corps d'embase (156) et la lumière du tube de cathéter (105) dans une position prête à l'emploi ; et
fixer une extension d'embase (158) ayant un corps (164) avec une poche (190) à l'embase de cathéter (102) en plaçant au moins une partie du corps d'embase (156) et du support en selle (266) dans la poche (190) et en empêchant l'embout de cathéter (102) de se déplacer par rapport à l'extension d'embase (158)
et placer un adaptateur à languettes (160) sur au moins une partie de la surface supérieure (156a) de l'embase de cathéter (102), l'adaptateur à languettes (160) ayant deux parois latérales (216, 216) définissant une largeur supérieure à la largeur du corps d'embase (156), dans lequel l'adaptateur à languettes (160) peut être séparé de l'embase de cathéter (102),
**caractérisé en ce que**
l'embase de cathéter (102) comprend un support en selle (266) ayant des surfaces de paroi définissant une largeur supérieure à la largeur du corps d'embase (156), dans lequel le support en selle (266) est fixé au corps d'embase (156) et l'adaptateur à languettes (160) est fixé au support en selle (266).
